# EUROPÄISCHE PATENTSCHRIFT

(11) **EP 1 144 355 B1**
(45) Veröffentlichungstag und Bekanntmachung des Hinweises auf die Patenterteilung: **08.12.2004**
(21) Anmeldenummer: 00902574.3
(22) Anmeldetag: 10.01.2000
(51) Int. Cl.: C07C 69/63, C08F 293/00, C08F 8/30, C08F 4/00

(54) **ALPHA-HALOGENCARBONSÄUREESTER MIT MEHRWERTIGEN ALKOHOLEN ALS INITIATOREN FÜR ATRP**
ALPHA-HALOGENATED ACID ESTERS WITH POLYVALENT ALCOHOLS AS ATOM TRANSFER RADICAL POLYMERIZATION INITIATORS
ESTERS D'ACIDE CARBOXYLIQUE ALPHA-HALOGENE AVEC DES ALCOOLS POLYVALENTS UTILISES COMME INITIATEURS POUR POLYMERISATION RADICALAIRE A TRANSFERT D'ATOME

(30) Priorität: 21.01.1999 CH 10799
(43) Veröffentlichungstag der Anmeldung: 17.10.2001
(73) Patentinhaber: Ciba Specialty Chemicals Holding Inc., 4057 Basel (CH)
(72) Erfinder: MÜHLEBACH, Andreas, CH-5070 Frick (CH); RIME, François, CH-2800 Delémont (CH)
(86) Internationale Anmeldenummer: PCT/EP2000/000097
(87) Internationale Veröffentlichungsnummer: WO 2000/043344

(56) Entgegenhaltungen:
- DE-A- 2 232 136
- KRZYSZTOF MATYJASZEWSKI ET AL.: "Synthesis and Characterization of Star Polymers with Varying Arm Number,Length, and Composition from Organic and Hydrid Inorganic/Organic Multifunctional Initiators" MACROMOLECULES., Bd. 32, Nr. 20, 1999, Seiten 6526-6535, XP000905555 AMERICAN CHEMICAL SOCIETY. EASTON., US ISSN: 0024-9297
- JIRO UEDA ET AL.: "Multifunctional Initiators for the Ruthenium-Mediated Living Radical Polymerization of Methyl Methacrylate:Di- and Trifunctional Dichloroacetates for Synthesis of Multiarmed Polymers" MACROMOLECULES., Bd. 31, Nr. 3, 10. Februar 1998 (1998-02-10), Seiten 557-562, XP000735814 AMERICAN CHEMICAL SOCIETY. EASTON., US ISSN: 0024-9297
- G.E.MCCASLAND ET AL.: "New Esters of Pentaerythritol" JOURNAL OF THE AMERICAN CHEMICAL SOCIETY., Bd. 74, Nr. 2, 22. Januar 1952 (1952-01-22), Seiten 564-565, XP002137151 AMERICAN CHEMICAL SOCIETY, WASHINGTON, DC., US ISSN: 0002-7863
- THOMAS ZIEGLER: "Preparation of Some Fully Chloroacetylated Glycopyranosyl Bromides:Useful Intermediates for the Synthesis of Base- and Hydrogenolysis-Sensitive Glycosides" LIEBIGS ANNALEN DER CHEMIE., Nr. 11, November 1990 (1990-11), Seiten 1125-1131, XP002137152 VERLAG CHEMIE GMBH. WEINHEIM., DE ISSN: 0170-2041

## Beschreibung

Gegenstand der vorliegenden Erfindung sind α-Halogencarbonsäureester mit mehrwertigen Alkoholen, welche als Initiatoren für ATRP verwendbar sind, Verfahren zur Herstellung dieser Initiatoren, mit den Initiatoren herstellbare Polymere oder Copolymere, Zusammensetzungen mit diesen Polymeren oder Copolymeren, Verfahren zu ihrer Herstellung und ihre Verwendung zur Herstellung von Polymeren oder Blockcopolymeren, worin die Endgruppe •X durch eine offenkettige oder cyclische Gruppe R'R"N-O• ersetzt ist.

Die radikalische Atomtransferpolymerisation (ATRP: Atom Transfer Radical Polymerization) ist ein seit längerem bekanntes, besonders geeignetes Polymerisationsverfahren zur Herstellung von "lebenden" Polymeren, Blockcopolymeren, Pfropfcopolymeren usw. mit einer niedrigen Polydispersität und weitgehend vorherbestimmbaren Molekulargewichten.

Trotz ihrer offensichtlichen Vorteile ist für solche Polymerisationsverfahren die geringe Auswahl an geeigneten Initiatoren nachteilig, die sich für die Herstellung von verzweigten Polymerstrukturen eignen. Die bekannten Polymerisationsinitiatoren, welche z. B. in der

WO 96/30421 beschrieben sind, z. B. 2-Chlor- oder 2-Bromessigsäure oder 2-Chlor- oder 2-Bromoisobuttersäure, führen zu linearen, aber nicht zu verzweigten Strukturen der Polymerkette und erlauben daher nur eine geringe strukturelle Variation der erhältlichen Polymere.

Der vorliegenden Erfindung liegt die Aufgabe zugrunde, verzweigte Polymerstrukturen, z. B. Sternpolymere, Dendrimere, Kammpolymere usw., unter Verwendung von geeigneten Polymerisationsinitiatoren herzustellen. Diese Aufgabe wird durch die vorliegende Erfindung gelöst, welche die Herstellung von Polymeren oder Blockcopolymeren unter Verwendung von α-Halogencarbonsäureestern betrifft, die durch einfache Acylierungsverfahren mit mehrwertigen Alkoholen herstellbar sind.

Gegenstand der Erfindung sind ein Polymer oder Blockcopolymer der Formel: 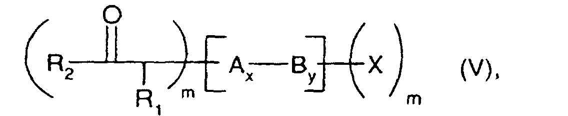 worin
- R₁: Wasserstoff, C₁-C₄-Alkyl, Cyan, Phenyl oder C₁-C₄-Alkylphenyl;
- R₂: den Rest eines acylierten, verzweigten, dreiwertigen Alkohols, den Rest eines vollständig oder partiell acylierten, linearen oder verzweigten, vierwertigen Alkohols, den Rest eines vollständig oder partiell acylierten, linearen, fünf- oder sechswertigen Alkohols, den Rest einer vollständig oder partiell acylierten, linearen oder cyclischen C₄-C₆-Aldose oder C₄-C₆-Ketose oder den Rest eines vollständig oder partiell acylierten Disaccharids;
- A und B: Polymerblöcke aus ethylenisch ungesättigten Monomereneinheiten;
- x und y: die Anzahl der Monomereneinheiten in den Blöcken A und B, wobei ein Wert von x und y null und der andere Wert ganze Zahlen grösser als null oder beide Werte x und y ganze Zahlen grösser als null betragen;
- X: Chlor, Brom oder lod; und
- m: ganze Zahlen von drei bis sechs bedeuten.

Ebenfalls Gegenstand der Erfindung ist ein Verfahren zur Herstellung eines Polymeren oder Blockcopolymeren (V), worin R₁, R₂, A, B, X, x, y und m die genannten Bedeutungen haben, welches dadurch gekennzeichnet ist, dass man durch radikalische Atomtransferpolymerisation (ATRP) in Gegenwart des Polymerisationsinitiators α-Halogencarbonsäureester (I) 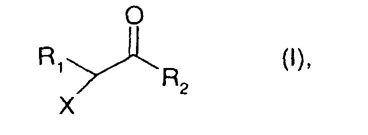 worin
R₁ Wasserstoff, C₁-C₄-Alkyl, Cyan, Phenyl oder C₁-C₄-Alkylphenyl;
X Chlor, Brom oder lod; und
R₂ den Rest eines acylierten, verzweigten, dreiwertigen Alkohols, den Rest eines vollständig oder partiell acylierten, linearen oder verzweigten, vierwertigen Alkohols, den Rest eines vollständig oder partiell acylierten, linearen, fünf- oder sechswertigen Alkohols, den Rest einer vollständig oder partiell acylierten, linearen oder cyclischen C₄-C₆-Aldose oder
C₄-C₆-Ketose oder den Rest eines vollständig oder partiell acylierten Disaccharids darstellen, und eines oxydierbaren Übergangsmetallkomplexkatalysators den Polymerblöcken A und B zugrundeliegende aliphatische Monomere mit Ethylengruppen einer Polymerisationsreaktion unterwirft.

Die in der Beschreibung der vorliegenden Erfindung verwendeten Begriffe und Bezeichnungen sind vorzugsweise wie folgt definiert:
C₁-C₄-Alkyl ist Methyl, Ethyl, n- oder Isopropyl oder n-, sek.- oder tert.-Butyl.
C₁-C₄-Alkylphenyl ist vorzugsweise p-Methylphenyl.
R₁ bildet vorzugsweise mit dem α-C-Atom eine 2-Haloacylgruppe, z.B. 2-Halo-C₃-C₄-alkanoyl, z.B. 2-Halopropionyl, 2-Halo-n-butyryl oder 2-Halo-isobutyryl, z.B. 2-Chlor- oder 2-Brompropionyl oder α-Chlor- oder α-Bromisobutyryl, oder eine α-Halophenylacetylgruppe, z.B. α-Chlor- oder α-Bromphenylacetat.

Der Rest R₂ eines acylierten, verzweigten, dreiwertigen Alkohols leitet sich vorzugsweise von 1,3,5-Trihydroxybenzol oder Trimethylolethan ab und ist z.B. eine Gruppe der Partialformel 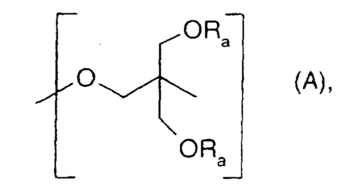 worin Rₐ α-Haloacyl bedeutet. Rₐ mit der Bedeutung α-Haloacyl stellt vorzugsweise identi- 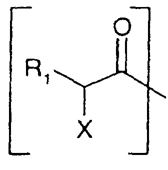 sche Gruppen der Partialformel dar, z.B. α-Halo-C₃-C₄-alkanoyl oder α-Halophenylacetyl, z.B. α-Chlorpropionyl, α-Brompropionyl oder α-Chlorphenylacetyl. Der Rest R₂ eines vollständig oder partiell acylierten, linearen vierwertigen Alkohols leitet sich z.B. von Erythrit und seinen 3 isomeren Formen ab, z. B. D-, L- und Mesoerythrit.

Vorzugsweise leitet sich der Rest R₂ von einem vollständig oder partiell acylierten, verzweigten vierwertigen Alkohol ab, z.B. von Pentaerythrit, und ist z.B. eine Gruppe der Partialformel 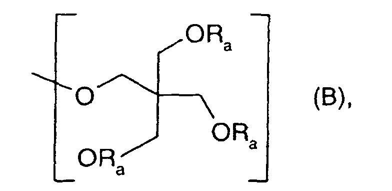 worin Rₐ α-Haloacyl mit den genannten Bedeutungen darstellt.

Der Rest eines vollständig oder partiell acylierten, linearen, fünf- oder sechswertigen Alkohols ist beispielsweise von linearen Pentiten, wie D(+)- und L(-)-Arabit, Adonit oder Xylit, oder linearen Hexiten, wie D-Sorbit, D-Mannit oder Dulcit, abgeleitet, deren Hydroxygruppen vollständig oder partiell durch Rₐ (= α-Haloacyl) substituiert sind.

Der Rest einer vollständig oder partiell acylierten, linearen oder cyclischen C₄-C₆-Aldose oder C₅-C₆-Ketose ist beispielsweise von C₄-Aldosen, wie D(-)- und L(+)-Erythrose oder D(-)-und L(+)-Threose, C₅-Aldosen, wie D(-)- und L(+)-Arabinose, D(-)-Ribose oder D(+)-Xylose, C₆-Aldosen, wie D(+)-Glucose, D(+)-Mannose oder D(+)-Galactose, oder einer C₆-Ketose, wie Fructose oder L(-)-Sorbose, und deren epimeren Formen abgeleitet, worin die Hydroxygruppen ebenfalls vollständig oder partiell durch Rₐ (=α-Haloacyl) substituiert sind.

Der Rest eines vollständig oder partiell acylierten Disaccharids leitet sich beispielsweise von Saccharose, Milchzucker oder Maltose ab, deren Hydroxygruppen ebenfalls vollständig oder partiell durch Rₐ (= α-Haloacyl) substituiert sind.

Vorzugsweise verwendet man α-Halogencarbonsäureester (I), worin
- R₁: C₁-C₃-Alkyl oder Phenyl;
- X: Chlor oder Brom und
- R₂: den Rest eines acylierten, verzweigten, dreiwertigen Alkohols, z.B. den durch Rₐ acylierten Rest von 1,3,5-Trihydroxybenzol oder Trimethylolethan, den Rest eines vollständig oder partiell acylierten, linearen oder verzweigten, vierwertigen Alkohols, z.B. den durch Rₐ vollständig acylierten Rest von Pentaerythrit, oder den Rest eines vollständig oder partiell acylierten, linearen, fünf- oder sechswertigen Alkohols darstellen, und Isomere dieser Verbindungen.

In diesen bevorzugten Ausführungsformen hat Rₐ mit der Bedeutung α-Haloacyl insbesondere die Bedeutung α-Chlorpropionyl und α-Brompropionyl.

Eine besonders bevorzugte Ausführungsform betrifft α-Halogencarbonsäureester der Formel 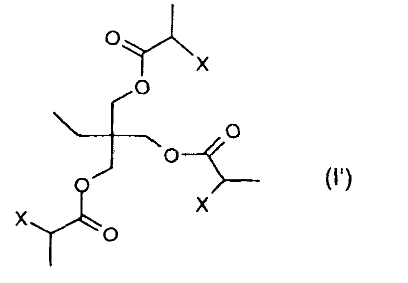 oder der Formel 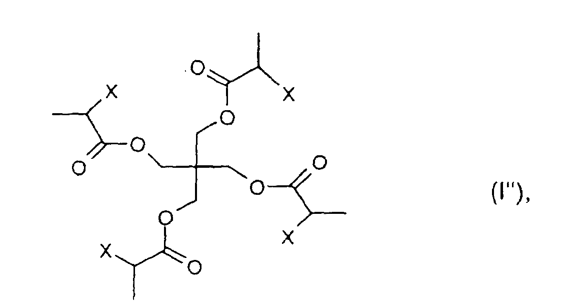 worin X Brom oder lod bedeuten.

α-Halogencarbonsäureester (I), worin R₁, R₂ und X die weiter vorn genannten Bedeutungen haben, werden hergestellt, indem man eine α-Halogencarbonsäure der Formel 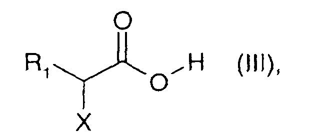 oder ein reaktionsfähiges, funktionelles Säurederivat davon mit einem Alkohol

HO-R₂' (IV),

oder einem reaktionsfähigen Alkoholderivat umsetzt, worin R₂' mit der OH-Gruppe einen verzweigten, dreiwertigen Alkohol, einen linearen oder verzweigten, vierwertigen Alkohol, einen linearen, fünf- oder sechswertigen Alkohol, eine lineare oder cyclische C₄-C₆-Aldose oder C₄-C₆-Ketose oder ein Disaccharid darstellt, und Isomere dieser Verbindungen.

Der Begriff isomere Formen umfasst die in der Chemie der Zuckeralkohole und Kohlehydrate bekannten Isomerieformen, z.B. die optisch reinen Stereoisomere (Antipoden), Diastereomere oder Epimere oder racemischen Gemische.

Zur Herstellung des α-Halogencarbonsäureesters (I) wendet man die üblichen Veresterungsmethoden an, indem man z.B. die Äquivalente eines reaktionsfähigen, funktionellen Säurederivates der α-Halogencarbonsäure (II), z.B. eines Säurehalogenids, z.B. des Säurechlorides, welche der Wertigkeit des Alkohols (III) entsprechen, mit,diesem Alkohol umsetzt, oder die α-Halogencarbonsäure (II) mit den Äquivalenten eines reaktionsfähigen, funktionellen Derivates des Alkohols (III), z.B. einem Ester dieses Alkohols, z.B. einem Halogenid, z.B. Chlorid, oder einem Sulfonsäureester des Alkohols, z.B. dem p-Toluolsulfonsäureester, umsetzt.

Der Begriff Polymer umfasst Oligomere, Cooligomere, Polymere oder Copolymere, beispielsweise Blockcopolymere, Multiblockcopolymere, sternförmige, gradient, random, verzweigte und dendritic Copolymere und Pfropfcopolymere. Die Copolymerblöcke A und B enthalten mindestens zwei wiederkehrende Struktureinheiten aus polymerisierbaren, aliphatischen Monomeren mit mindestens einer oder mehreren olefinischen Doppelbindungen.

Solche polymerisierbaren, aliphatischen Monomere mit einer olefinischen Doppelbindung sind beispielsweise aus der Gruppe ausgewählt enthaltend Styrole, Acrolein, Acrylsäure oder Methacrylsäure oder Salze davon, Acrylsäure- oder Methacrylsäureanhydride, Acrylsäure- oder Methacrylsäure-C₁-C₂₄-alkylester, Acrylsäure- oder Methacrylsäure-mono- oder - di-C₁-C₄-alkylamino-C₂-C₄-alkylester, Acrylsäure- oder Methacrylsäure-hydroxy-C₂-C₄-alkylester, Acrylsäure- oder Methacrylsäure-(C₁-C₄-alkyl)₃silyloxy-C₂-C₄-alkylester, Acrylsäureoder Methacrylsäure-(C₁-C₄-alkyl)₃silyl-C₂-C₄-alkylester, Acrylsäure- oder Methacrylsäureheterocyclyl-C₂-C₄-alkylester, Acryl- oder Methacrylsäureester mit Poly-C₂-C₄-alkylenglycolestergruppen, die ihrerseits durch substituierte C₁-C₂₄-Alkoxygruppen verestert sein können, Acrylsäure- oder Methacrylsäureamide, Acrylsäure- oder Methacrylsäure-monooder -di-C₁-C₄-alkylamide, Acrylsäure- oder Methacrylsäure-amino-C₂-C₄-alkylamide und Acrylonitril.

Geeignete Styrole können an der Phenylgruppe durch ein bis drei Substituenten aus der Gruppe enthaltend Hydroxy, C₁-C₄-Alkoxy, z.B. Methoxy oder Ethoxy, Halogen, z.B. Chlor, Amino und C₁-C₄-Alkyl, z.B. Methyl oder Ethyl, substituiert sein.

Geeignete Salze der Acrylsäure oder Methacrylsäure sind z.B. (C₁-C₄-Alkyl)₄ammoniumoder (C₁-C₄-Alkyl)₃NH-salze, z.B. das Tetramethyl-, Tetraethyl-, Trimethylammonium- oder Triethylammoniumsalz, das Trimethyl-2-hydroxyethylammonium- oder Triethyl-2-hydroxyethylammoniumsalz, das Dimethyl-2-hydroxyethylammonium- oder Diethyl-2-hydroxyethylammoniumsalz.

Geeignete Acrylsäure- oder Methacrylsäure-C₁-C₂₄-alkylester sind beispielsweise durch Methyl, Ethyl, n-Butyl, Isobutyl, tert.-Butyl, 2-Ethylhexyl, Isobornyl, Isodecyl, Lauryl, Myristyl, Stearyl oder Behenyl verestert.

Beispiele für Acrylsäure- und Methacrylsäure-mono- oder -di-C₁-C₄-alkylamino-C₂-C₄-alkylester sind Acrylsäure- oder Methacrylsäure-2-monomethylaminoethylester, Acrylsäure- oder Methacrylsäure-2-dimethylaminoethylester oder die entsprechenden 2-Monoethylaminoethyl- oder 2-Diethylaminoethylester sowie der Acrylsäure- oder Methacrylsäure-2-tert.-butylaminoethylester.

Beispiele für Acrylsäure- und Methacrylsäure-hydroxy-C₂-C₄-alkylester sind Acrylsäure- oder Methacrylsäure-2-hydroxyethylester (HEA, HEMA) oder Acrylsäure- oder Methacrylsäure-2-hydroxypropylester (HPA, HPMA).

Beispiele für Acrylsäure- und Methacrylsäure-silyloxy-C₂-C₄-alkylester sind Acrylsäure- oder Methacrylsäure-2-trimethylsilyloxyethylester (TMS-HEA, TMS-HEMA). Beispiele für Acrylsäure- oder Methacrylsäure-(C₁-C₄-alkyl)₃silyl-C₂-C₄-alkylester sind Acrylsäure- oder Methacrylsäure-2-trimethylsilylethylester oder Acrylsäure- oder Methacrylsäure-3-trimethylsilyl-n-propylester.

Acryl- oder Methacrylsäureester mit Poly-C₂-C₄-alkylenglycolestergruppen, die ihrerseits durch substituierte C₁-C₂₄-Alkoxygruppen verestert sein können, entsprechen der Formel: 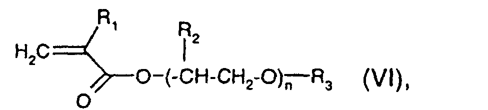 worin R₁ und R₂ unabhängig voneinander Wasserstoff oder Methyl und R₃ C₁-C₂₄-Alkyl bedeuten, z.B. Methyl, Ethyl, n- oder Isopropyl, n-, Iso-, oder tert.-Butyl, n- oder Neopentyl, Lauryl, Myristyl oder Stearyl, oder Aryl-C₁-C₂₄-alkyl, z.B. Benzyl oder Phenyl-n-nonyl, sowie C₁-C₂₄-alkylaryl oder C₁-C₂₄-alkylaryl-C₁-C₂₄-alkyl.

Beispiele für Acrylsäure- und Methacrylsäure-heterocyclyl-C₂-C₄-alkylester sind Acrylsäureoder Methacrylsäure-2-(N-morpholinyl, 2-pyridyl, 1-imidazolyl, 2-oxo-1-pyrrolidinyl, 4-methylpiperidin-1-yl oder 2-oxoimidazolidin-1-yl)-ethylester.

Beispiele für die genannten Acrylsäure- oder Methacrylsäure-mono- oder -di-C₁-C₄-alkylamide, Acrylsäure- oder Methacryl-di-C₁-C₄-alkylamino-C₂-C₄-alkylamide oder Acrylsäureoder Methacrylsäureamino-C₂-C₄alkylamide sind N,N-Dimethylacrylamid, N,N-Dimethyl-(meth)acrylamid, 2-(N,N-Dimethylaminoethyl)-acrylamid, 2-(N,N-Dimethylaminoethyl)-methacrylamid, 2-Aminoethylacrylamid und 2-Aminoethylmethacrylamid.

Die Indizes x und y definieren die Anzahl der Monomereneinheiten in den Blöcken A und B, wobei ein Wert von x und y null und der andere Wert ganze Zahlen grösser als null oder beide Werte x und y ganze Zahlen grösser als null betragen. Für x und y ist ein Zahlenbereich von zwei bis 1000 bevorzugt.

In einem Blockcopolymer (V) beträgt der bevorzugte Molekulargewichtsbereich der Blöcke A und B ca. 1 000 bis 100 000, insbesondere ca. 1 000 bis 50 000. Ein ganz besonders bevorzugter Molekulargewichtsbereich beträgt ca. 2 000 bis 15 000.

Eine besonders bevorzugte Ausführungsform der Erfindung betrifft ein Blockcopolymer (V), worin
- R₁: C₁-C₃-Alkyl oder Phenyl;
- X: Chlor oder Brom und
- R₂: den Rest eines acylierten, verzweigten, dreiwertigen Alkohols, den Rest eines acylierten, linearen oder verzweigten, vierwertigen Alkohols oder den Rest eines vollständig oder partiell acylierten, linearen, fünf- oder sechswertigen Alkohols;
- A und B: Polymerblöcke aus ethylenisch ungesättigten Monomereneinheiten;
- x und y: ganze Zahlen grösser als null und die Anzahl der Monomereneinheiten in den Blöcken A und B darstellen; und
- m: drei oder vier bedeuten.

Die Erfindung betrifft sämtliche Polymere oder Blockcopolymere, die unter Verwendung von α-Halogencarbonsäureestern (I) und der ATRP-Methode herstellbar sind.

In einem Blockcopolymer (V) stellt X endständiges Chlor, Brom oder lod der Polymerkette dar. Man erhält diese endständige Gruppen bei Verwendung von Initiatoren nach der ATRP-Methode. Halogen als endständige Gruppe einer Polymerenkette kann nachteilig sein. Daher kann man in einem Folgeschritt Halogen durch andere geeignete endständige Gruppen ersetzen, die von TEMPO (= 2,2,6,6-TetraMethylPiperidyl-1-Oxide) und Derivaten davon abgeleitet sind und eine Struktur der folgenden Partialformel aufweisen: 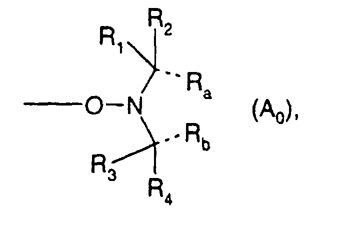 worin
einer von R₁ und R₂ C₁-C₇-Alkyl und der andere C₁-C₄-Alkyl oder durch C₁-C₄-Alkoxycarbonyl oder C₁-C₄-Alkoxy substituiertes C₁-C₄-alkyl bedeutet; oder
- R₁ und R₂: zusammen mit dem benachbarten C-Atom C₃-C₇-Cycloalkyl bedeuten;
- R₃ und R₄: die Bedeutungen von R₁ und R₂ haben;
- Rₐ: C₁-C₄-Alkyl, Cyan, C₁-C₄-Alkoxycarbonyl, C₁-C₄-Alkanoyloxy, C₁-C₄-Alkanoyloxy-C₁-C₄-alkyl, Carbamoyl, Mono- oder Di-C₁-C₄-alkylcarbamoyl, Mono- oder Di-2-hydroxyethylcarbamoyl, Amidino, 2-Imidazolyl, 1-Hydroxy-2-hydroxymethyl-2-propylcarbamoyl, oder 1,1-Dihydroxymethyl-2-hydroxycarbamoyl; und
- R_{b}: die Bedeutungen von Rₐ hat; oder
- Rₐ und R_{b}: zusammen eine bivalente Gruppe und eine aliphatische oder aromatische heterocyclische Gruppe mit 5, 6, 7 oder 8-Ringgliedern bilden, welche 1-3 zusätzliche Heteroatome aus der Gruppe Stickstoff, Sauerstoff und Schwefel enthalten kann.

Eine bevorzugte Ausführungsform umfasst eine Gruppe der Partialformel: 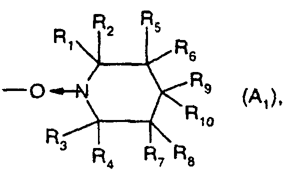 welche in 4-Stellung durch ein oder zwei Substituenten substituiert sein kann. In der Partialformel A₁ bedeuten
R₁, R₂, R₃ und R₄ C₁-C₄-Alkyl;
R₅, R₆, R₇ und R₈ Wasserstoff; und
einer der Reste R₉ und R₁₀ unabhängig voneinander Wasserstoff oder weitere Substituenten.

Repräsentative Beispiele für Gruppen der Partialformel A, sind die Gruppen 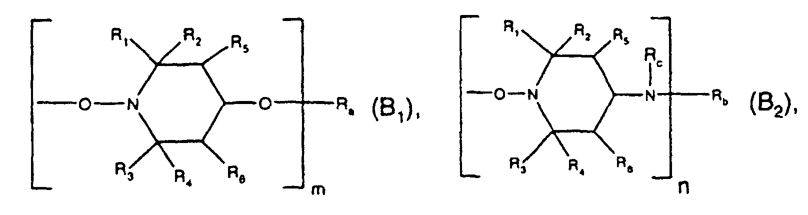 und 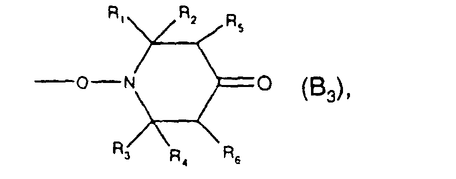 worin
m 1;
- Rₐ: Wasserstoff, C₁-C₁₈-Alkyl, welches durch ein oder mehrere Sauerstoffatome unterbrochen sein kann, 2-Cyanethyl, Benzoyl, Glycidyl, oder die Acylgruppe einer aliphatischen C₂-C₁₂-Carbonsäure, einer cycloaliphatischen C₇-C₁₅-Carbonsäure, einer a,b-ungesättigten C₃-C₅-Carbonsäure oder einer aromatischen C₇-C₁₅-Carbonsäure;
m 2;
- Rₐ: die bivalente Acylgruppe einer aliphatischen C₂-C₃₆-Dicarbonsäure;
n 1;
- R_{b}: C₁-C₁₂-Alkyl, C₅-C₇-Cycloalkyl, C₇-C₈-Aralkyl, C₂-C₁₈-Alkanoyl, C₃-C₅-Alkenoyl oder Benzoyl; und
- R_{c}: C₁-C₁₈-Alkyl, C₅-C₇-Cycloalkyl, C₂-C₈-Alkenyl, welches durch Cyan, Carbonyl oder eine Carbamidgruppe substituiert sein kann, Glycidyl, oder eine Gruppe der Teilformeln - CH₂CH(OH)-Z, -CO-Z oder -CONH-Z bedeuten, worin Z Wasserstoff, Methyl oder Phenyl bedeutet.

Ein weitere bevorzugte Ausführungsform bezieht sich auf eine Gruppe der Partialformel A₁, worin eine der Gruppen R₉ und R₁₀ Wasserstoff und die andere C₁-C₄-Alkanoyl oder C₁-C₄-Alkanoylamino bedeutet.

Ein weiterer Erfindungsgegenstand betrifft ein N → O substituiertes Polymer oder Blockcopolymer der Formel: 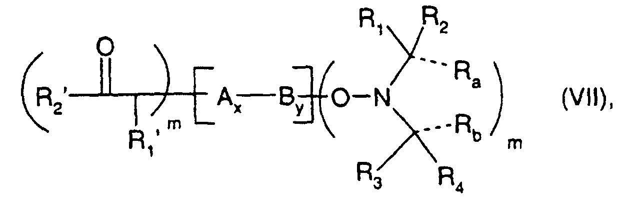 worin
- R₁': Wasserstoff, C₁-C₄-Alkyl, Cyan, Phenyl oder C₁-C₄-Alkylphenyl;
- R₂': den Rest eines acylierten, verzweigten dreiwertigen Alkohols, den Rest eines vollständig oder partiell acylierten, linearen oder verzweigten, vierwertigen Alkohols, den Rest eines vollständig oder partiell acylierten, linearen, verzweigten oder cyclischen, fünf- oder sechswertigen Alkohols, den Rest einer vollständig oder partiell acylierten, linearen oder cyclischen C₄-C₆-Aldose oder C₄-C₆-Ketose oder den Rest eines vollständig oder partiell acylierten Disaccharids;
- A und B: Polymerblöcke aus ethylenisch ungesättigten Monomereneinheiten;
- x und y: die Anzahl der Monomereneinheiten in den Blöcken A und B, wobei ein Wert von x und y null und der andere Wert ganze Zahlen grösser als null oder beide Werte x und y ganze Zahlen grösser als null betragen;
- m: ganze Zahlen von drei bis sechs bedeuten;
einer von R₁ und R₂ C₁-C₇-Alkyl und der andere C₁-C₄-Alkyl oder durch C₁-C₄-Alkoxycarbonyl oder C₁-C₄-Alkoxy substituiertes C₁-C₄-Alkyl bedeutet; oder
- R₁ und R₂: zusammen mit dem benachbarten C-Atom C₃-C₇-Cycloalkyl bedeuten;
- R₃ und R₄: die Bedeutungen von R₁ und R₂ haben;
- Rₐ: C₁-C₄-Alkyl, Cyan, C₁-C₄-Alkoxycarbonyl, C₁-C₄-Alkanoyloxy, C₁-C₄-Alkanoyloxy-C₁-C₄-alkyl, Carbamoyl, Mono- oder Di-C₁-C₄-alkylcarbamoyl, Mono- oder Di-2-hydroxyethylcarbamoyl, Amidino, 2-Imidazolyl, 1-Hydroxy-2-hydroxymethyl-2-propylcarbamoyl, oder 1,1-Dihydroxymethyl-2-hydroxycarbamoyl; und
- R_{b}: die Bedeutungen von Rₐ hat; oder
- Rₐ und R_{b}: zusammen eine bivalente Gruppe und eine aliphatische oder aromatische heterocyclische Gruppe mit 5, 6, 7 oder 8-Ringgliedern bilden, welche 1-3 zusätzliche Heteroatome aus der Gruppe Stickstoff, Sauerstoff und Schwefel enthalten kann.

Das Polymerisationsverfahren ist in Gegenwart von Wasser oder einem organischen Lösungsmittel oder Mischungen davon durchführbar. Es lassen sich zusätzliche Cosolventien oder Tenside, zum Beispiel Glycole oder Ammoniumsalze von Carbonsäuren, dem Reaktionsgemisch hinzufügen. Die Menge an Lösungsmittel ist möglichst gering zu halten. Das Reaktionsgemisch kann die oben erwähnten Monomere oder Oligomere in einer Konzentration von 1,0 bis 99,9 Gewichtsprozent enthalten, vorzugsweise 5,0 bis 99,9 Gewichtsprozent, besonders bevorzugt 50,0 bis 99,9 Gewichtsprozent, bezogen auf den Monomerengehalt im Polymerisat.

Geeignete organische Lösungsmittel sind Alkane (Hexan, Heptan, Octan, Isooctan), Kohlenwasserstoffe (Benzol, Toluol, Xylol), halogenierte Kohlenwasserstoffe (Chlorbenzol), Alkanole (Methanol, Ethanol, Ethylenglycol, Ethylenglycolmonomethylether), Ester (Essigester) oder Ether (Diethyl-, Dibutylether, Ethylenglycoldimethylether, Tetrahydrofuran) oder Mischungen davon.

Bei Verwendung von Wasser als Lösungsmittel kann man dem Reaktionsgemisch ein mit Wasser mischbares oder hydrophiles Lösungsmittel hinzufügen. Dabei ist darauf zu achten, dass während der Polymerisationsreaktion das Reaktionsgemisch in einer einzigen homogenen Phase bleibt und keine Ausfällung oder Phasentrennung stattfindet. Geeignete Cosolventien sind aus der Gruppe der aliphatischen Alkohole, Glycole, Ether, Glycolether, Pyrrolidine, N-Alkylpyrrolidinone, Polyethylenglycole, Polypropylenglycole, Amide, Carbonsäuren und deren Salze, Ester, Organosulfide, Sulfoxide, Sulfone, Alkoholderivate, Hydroxyetherderivate, z.B. Butylcarbitol oder Cellosolve, Aminoalkohole, Ketone, Derivate und Mischungen davon ausgewählt, z.B. Methanol, Ethanol, Propanol, Dioxan, Ethylenglycol, Propylenglycol, Diethylenglycol, Glycerin, Dipropylenglycol, Tetrahydrofuran oder andere wasserlösliche oder mit Wasser mischbare Lösungsmittel oder Mischungen davon.

Hydrophile Monomere, Polymere und Copolymere lassen sich aus dem Reaktionsgemisch unter Anwendung üblicher Verfahren zum Beispiel durch Destillation, Ausfällung, Extraktion, Änderung des pH-Bereichs oder andere übliche Trennmethoden abtrennen. Der Temperaturbereich für die Polymerisationsreaktion beträgt ca. 50°C bis ca. 180°C, vorzugsweise ca. 80°C bis 150°C.

Der im ATRP-Verfahren verwendbare oxydierbare Übergangsmetallkomplexkatalysator liegt in Form eines oxydierbaren Komplexions in der niederen Stufe eines Redoxsystems vor. Bevorzugte Beispiele solcher Redoxsysteme werden von Elementen der Gruppen V(B), VI(B), VII(B), VIII, IB und IIB des Periodensystems gebildet, z.B. Redoxsystemen von Cu⁺/Cu²⁺, Cu⁰/Cu⁺, Fe⁰/Fe²⁺, Fe²⁺/Fe³⁺, Cr^{2+/}Cr³⁺, Co⁺/Co²⁺, Co²⁺/Co³⁺, Ni⁰/Ni⁺, Ni⁺/Ni²⁺, Ni²⁺/Ni³⁺, Mn⁰/Mn²⁺, Mn²⁺/Mn³⁺, Mn³⁺/Mn⁴⁺ oder Zn⁺/Zn²⁺. Das Übergangsmetall bzw. Übergangsmetallkation im oxydierbaren Übergangsmetallkomplexkatalysator wird aus der niedrigeren Oxydationsstufe in eine höhere überführt. In einer bevorzugten Ausführungsform des Verfahrens wird ein Cu(I)-Komplexkatalysatorsalz in die entsprechende Cu(II)-Oxydationsstufe überführt.

Der im ATRP-Verfahren verwendbare oxydierbare Übergangsmetallkomplexkatalysator ist in einer separaten Vorstufe oder vorzugsweise in situ aus den Liganden und einem Metallsalz, z.B. Cu(I)Cl, herstellbar, welches anschliessend in die Komplexverbindung durch Zusatz des Ligandenbildners, z.B. Ethylendiamin, EDTA, Me₆TREN oder PMDETA, überführt wird.

Die ionischen Ladungen werden von anionischen Liganden ausgeglichen, welche aus der Komplexchemie der Übergangsmetalle bekannt sind, z.B. Hydridionen (H⁻) oder Anionen von anorganischen oder organischen Säuren, z.B. F⁻, Cl⁻, Br⁻ oder I⁻, Fluorkomplexen des Typs BF₄⁻, PF₆⁻, SbF₆⁻ oder AsF₆⁻, Anionen von Sauerstoffsäuren, Alkoholate oder Acetylide oder Anionen vom Typ des Cyclopentadienanions.

Anionen von Sauerstoffsäuren sind z.B. Sulfat, Phosphat, Perchlorat, Perbromat, Periodat, Antimonat, Arsenat, Nitrat, Carbonat, Anionen von C₁-C₈-Carbonsäuren, z.B. Format, Acetat, Propionat, Butyrat, Benzoat, Phenylacetat, Mono-, Di- oder Trichloracetat oder -fluoracetat, Sulfonate, z.B. Mesylat, Ethan-, Propan- oder n-Butansulfonat, Trifluormethansulfonat (Triflat) oder Benzol- oder Benzylsulfonat, welche durch C₁-C₄Alkyl, C₁-C₄-Alkoxy oder Halogen, insbesondere Fluor, Chlor oder Brom, substitutiert sein können, z.B. Tosylat, Brosylat, p-Methoxy- oder p-Ethoxybenzolsulfonat, Pentafluorbenzolsulfonat oder 2,4,6-Triisopropylbenzolsulfonat, Phosphonate, z.B. Methyl-, Ethyl-, n-Propyl- oder n-Butylphosphonat, Phenylphosphonat, p-Methylphenylphosphonat oder Benzylphosphonat, sowie C₁-C₁₂-alkoholate, z.B. Methanolat oder Ethanolat.

Neutrale und anionische Liganden können bis zur bevorzugten Koordinationszahl, insbesondere vier, fünf und sechs, vorhanden sein. Negative Gesamtladungen werden von Kationen, z. B. monovalenten Kationen, z.B. Na⁺, K⁺, NH₄⁺ oder (C₁-C₄Alkyl)₄N⁺ ausgeglichen.

Geeignete neutrale Liganden sind aus der Komplexchemie der Übergangsmetalle bekannt. Sie sind mit dem Koordinationszentrum unter Ausprägung von verschiedenen Bindungstypen koordiniert, z.B. σ-, π-, µ-, η-Bindungen oder Kombinationen davon bis zur bevorzugten Koordinationszahl des Komplexkations. Geeignete Liganden sind aus der Gruppe ausgewählt enthaltend Aquo (H₂O), Amino, Stickstoff, Kohlenmonoxid, Nitrosyl, Phosphine, z.B. (C₆H₅)₃P, (i-C₃H₇)₃P, (C₅H₉)₃P oder (C₆H₁₁)₃P, Amine, z.B. Ethylendiamin, Ethylendiaminotetraacetat (EDTA), N,N-Dimethyl-N',N'-bis(2-dimethylaminoethyl)ethylendiamin (Me₆TREN), Catechol, N,N'-Dimethyl-1,2-phenyldiamin, 2-(Methylamino)phenol, 3-(Methylamino)-2-butanol, N,N'-bis(1,1-Dimethylethyl)-1,2-ethandiamin oder N,N,N',N",N"-Pentamethyldiethyltriamin (PMDETA), C₁-C₈-Glycole oder Glyceride, z.B. Ethylen- oder Propylenglycol oder Derivate davon, z.B. Di-, Tri- oder Tetraglyme, und monodentate oder bidentate heterocyclische e⁻-Donor Liganden.

Heterocyclische e⁻-Donorliganden sind beispielsweise von unsubstituierten oder substituierten Heteroarenen abgeleitet aus der Gruppe enthaltend Furan, Thiophen, Pyrrol, Pyridin, bis-Pyridin, Picolylimin, γ-Pyran, γ-Thiopyran, Phenanthrolin, Pyrimidin, bis-Pyrimidin, Pyrazin, Indol, Coumarin, Thionaphthen, Carbazole, Dibenzofuran, Dibenzothiophen, Pyrazol, Imidazol, Benzimidazol, Oxazol, Thiazol, bis-Thiazol, Isoxazol, Isothiazol, Chinolin, Bischinolin, Isochinolin, Bisisochinolin, Acridin, Chroman, Phenazine, Phenoxazine, Phenothiazine, Triazine, Thianthren, Purin, bis-Imidazol und Bisoxazol.

Anschliessend an die Polymerisationsreaktion kann man das Polymerisat (V) isolieren oder dieses mit einer N → O Verbindung der Formel 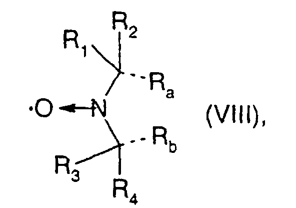 welche der Gruppe der Partialformel A₀ entspricht und worin R₁ - R₄ und Rₐ und R_{b} die weiter vorn genannten Bedeutungen haben, vorzugsweise in-situ, umsetzen, und das N → O substituierte Polymer oder Blockcopolymer (VII) herstellen. Das Isolieren des Polymerisats kann z.B. nach bekannten Methoden erfolgen, z.B. Destillieren und Abfiltrieren von nicht umgesetzten Monomeren.

Die Verwendung eines Polymeren oder Blockcopolymeren (V) zur Herstellung von Polymeren oder Blockcopolymeren (VII), worin •X durch eine offenkettige oder cyclische Gruppe R'R"N-O· ersetzt ist, ist ebenfalls Gegenstand der Erfindung.

Nach der Substitution des Polymerisats mit der N→O Verbindung (VIII) trennt man den Übergangsmetallkomplexkatalysator ab, dampft das Lösungsmittel ab oder fällt das durch die N→O Gruppe substituierte Polymer (VII) aus einer geeigneten flüssigen Phase aus, filtriert das Polymer ab und wäscht und trocknet anschliessend.

Die Eliminierung der Abgangsgruppe -X, z.B. Halogen, und die Substitution des Polymerisats mit der N → O Verbindung (VIII) erfolgt z.B. durch Auflösen des Polymerisats (V) in einem Lösungsmittel und Hinzufügen der N→O Verbindung (VIII). Die Umsetzung ist in einem Temperaturbereich von Raumtemperatur bis zur Siedetemperatur des Reaktionsgemisches durchführbar, vorzugsweise von Raumtemperatur bis 100°C.

Da die Polymerisation und anschliessende Derivatisierung mit einer N→O Verbindung (VIII) nach der ATRP-Methode die Merkmale einer "lebenden" Polymerization hat, kann man diese nach Belieben starten und beenden. Die verfahrensgemäss erhältlichen Blockcopolymere (V) und (VII) haben eine geringe Polydispersität. Vorzugsweise erhält man eine Polydispersität von 1,01 bis 2,2, vorzugsweise 1,01 bis 1,9, insbesondere 1,01 bis 1,5.

N → O Verbindungen (VIII) sind bekannt. Sie sind kommerziell erhältlich oder nach den in den U.S. Patentschriften 5,204,473 oder 4,581,429 und den darin zitierten Publikationen genannten Verfahren herstellbar.

Das ATRP-Verfahren und seine diversen Vorteile sind z.B. in der Publikation von K. Matyjaszewski in ACS Symp. Ser. Vol. 685 (1998), pg. 2-30, beschrieben. Die Polymere und Copolymere lassen sich nach üblichen Verfahren weiterverarbeiten und sind in den meisten Fällen ohne weitere Reinigungsschritte anwendbar. Dies ist vorteilhaft, wenn eine massstäbliche Vergrösserung von Ansätzen ("scale-up") im Hinblick auf eine industrielle Anwendung beabsichtigt ist.

Die Erfindung betrifft auch sämtliche N → O substituierten Polymere oder Blockcopolymere, die unter Verwendung von α-Halogencarbonsäureestern (I), einer N → O Verbindung der Formel VIII und der ATRP-Methode herstellbar sind.

Ein weiterer Erfindungsgegenstand betrifft eine Polymerzusammensetzung enthaltend ein Polymer oder Blockcopolymer (V), worin R₁, R₂, A, B, x, y und m die genannten Bedeutungen haben, und in Polymerzusammensetzungen enthaltene übliche Zusatzstoffe.

Ebenfalls Gegenstand der Erfindung sind Polymerzusammensetzungen, enthaltend ein Polymer oder Blockcopolymer (V) im Gemisch mit einem N → O substituierten Polymer oder Blockcopolymer (VII) und in Polymerzusammensetzungen enthaltene übliche Zusatzstoffe.

Solche Zusatzstoffe können in geringen Mengen hinzugesetzt werden, z.B. UV-Absorber oder Lichtschutzmittel, z.B. aus der Reihe der Hydroxyphenylbenzotriazole, Hydroxyphenylbenzophenone, Oxalamide oder Hydroxyphenyl-s-triazine. Besonders geeignet sind Lichtschutzmittel aus der Gruppe der sogenannten sterisch gehinderten Amine (HALS), z.B. vom 2-(2-Hydroxyphenyl)-1,3,5-triazin- oder 2-Hydroxyphenyl-2H-benzotriazol-Typ. Beispiele von Lichtschutzmitteln des 2-(2-Hydroxyphenyl)-1,3,5-triazin-Typs sind aus der Patentliteratur bekannt, z.B. US-A-4619956, EP-A-434608, US-A-5198498, US-A-5322868, US-A-5369140, US-A-5298067, WO-94/18278, EP-A-704437, GB-A-2297091 oder WO-96/28431.

Die Zusammensetzungen können noch weitere übliche Zusatzstoffe enthalten, z.B. Füllstoffe, z.B. Calciumcarbonat, Silicate, Glas oder Glasfasermaterial, Talkum, Kaolin, Mica, Bariumsulfat, Metalloxide und -hydroxide, Russ, Graphit, gepulvertes Holz sowie gepulvertes oder faserartiges Material von anderen Naturprodukten, synthetische Fasern, Weichmacher, Schmiermittel, Emulgatoren, Pigmente, Fliessmittel, Katalysatoren, optische Aufheller, Flammschutzmittel, Antistatika oder Treibmittel.

Die Zusammensetzung kann die genannten Polymere in Konzentrationen von ca. 0,01 bis 99,0 Gewichtsprozent, vorzugsweise 0,1 bis 95 Gewichtsprozent, insbesondere 1,0 bis 90,0 Gewichtsprozent, vor allem 5,0 bis 80,0 Gewichtsprozent, bezogen auf den Monomerengehalt der Zusammensetzung, enthalten.

Ein weiterer Erfindungsgegenstand ist eine Polymerzusammensetzung enthaltend
a) ein Polymer oder Blockcopolymer (V),
   worin R₁, R₂, A, B, x, y und m die weiter vorn genannten Bedeutungen haben; und
b) ein weiteres Polymer oder Oligomer der Formel

   Aₓ-B_{y} (IX),

   worin
   A und B Polymerblöcke aus ethylenisch ungesättigten Monomereneinheiten und x und y die Anzahl der Monomereneinheiten in den Blöcken A und B bedeuten, wobei ein Wert von x und y null und der andere Wert ganze Zahlen grösser als null oder beide Werte x und y Zahlen grösser als null betragen.

Ebenfalls Gegenstand der Erfindung ist eine Polymerzusammensetzung enthaltend
a') ein N → O substituiertes Polymer oder Blockcopolymer (VII) und
b') ein weiteres Polymer oder Oligomer (IX).

Die Zusammensetzungen können die genannten üblichen Zusatzstoffe und die Polymeroder Oligomerkomponenten a) und b), bzw. a') und b'), in Konzentrationen von ca. 0,01 bis 99,0 Gewichtsprozent, vorzugsweise 0,1 bis 95 Gewichtsprozent, insbesondere 1,0 bis 90,0 Gewichtsprozent, vor allem 5,0 bis 80,0 Gewichtsprozent, bezogen auf den Monomerengehalt der Zusammensetzung, enthalten.

Die Polymere und die Zusammensetzungen gemäss vorliegender Erfindung sind für unterschiedlichste technische Applikationen verwendbar, z.B. als Klebstoffe, Waschmittelhilfsstoffe, Detergentien, Dispergiermittel, Emulgatoren, Tenside, Entschäumungsmittel, Haftvermittler, Korrosionsinhibitoren, Viskositätsverbesserer, Schmiermittel, Fliessverbesserer, Verdicker, Vernetzungsmittel, als Zusatzstoffe für die Wasseraufbereitung, elektronische Materialien, Farben und Lacke, Beschichtungen, Tinten, Photoentwickler, "Superabsorbants", Kosmetika, Konservierungsmittel, oder als Biozide oder Modifikatoren und Hilfsstoffe für Asphalt, Textilien, Keramik und Holz.

### Beispiele

### Beispiel 1

Herstellung der Verbindung:

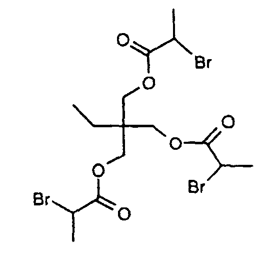

Ansatz:
26,84 g (0,2 Mol) 1,1,1-(Tris-hydroxymethyl)-propan (Fluka, purum); 136,4 g (0,6 Mol) 2-Brom-propanoyl-bromid (Fluka, pract. 95%); 47,5 g (0,6 Mol) Pyridin (Fluka, puriss. p.a.); 500 ml THF (Fluka, puriss. p.a.); 1500 ml Sulfierkolben mit Rückflusskühler und mechanischem Rührwerk.

Zu einer Lösung von 1,1,1-(tris-Hydroxymethyl)-propan und Pyridin in 320 ml THF wird unter Kühlung auf 10-15°C eine Lösung von 2-Brompropanoyl-bromid in 180 ml THF während 45 Min. zugetropft (leicht exotherme Reaktion). Danach wird während 3 h auf 60°C erwärmt und die Reaktionsmischung abgekühlt und filtriert. Man verdünnt mit 500 ml tert-Butylmethylether und extrahiert 2x mit je 150 ml Wasser bis zur neutralen Reaktion. Die organische Phase wird über Na₂SO₄ getrocknet, filtriert und im Rotationsverdampfer vollständig eingeengt. Rohausbeute: 116,95 g. Das Rohprodukt wird säulenchromatographisch (Kieselgel, Toluol als Eluiermittel) gereinigt: Ausbeute an Reinprodukt: 70,48 g (65%).

| Elementaranalyse: | | |
|---|---|---|
| C | H | Br |
| 33,69¹ | 4,30¹ | 44,47¹ |
| 33,63² | 4,22² | 44,60² |

| | | |
|---|---|---|
| ¹⁾berechnet; | | |
| ²⁾gefunden | | |

### Beispiel 2

Herstellung der Verbindung

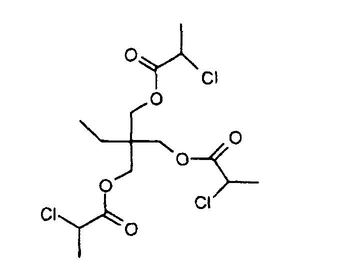

Ansatz:
26,84 g (0,2 Mol) 1,1,1-(tris-Hydroxymethyl)-propan (Fluka, purum); 76,18 g (0,6 Mol) 2-Chlorpropanoylchlorid (Fluka, pract. 97%); 47,5 g (0,6 Mol) Pyridin (Fluka, puriss. p.a.); 500 ml THF (Fluka, puriss. p.a.); 1500 ml Sulflerkolben mit Rückflusskühler und mechanischem Rührwerk.

Analog Beispiel 1 erhält man 66,32 g (82 %) des reinen Produkts.

| Elementaranalyse: | | |
|---|---|---|
| C | H | Cl |
| 44,41¹ | 5,71¹ | 26,21¹ |
| 44,09² | 5,34² | 26,45² |

| | | |
|---|---|---|
| ¹⁾berechnet; | | |
| ²⁾gefunden | | |

### Beispiel 3

Herstellung der Verbindung

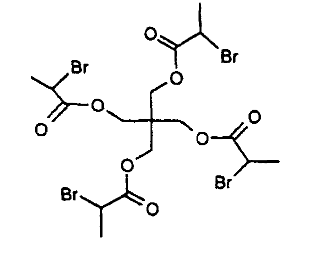

Ansatz:
27,20 g (0,2 Mol) Pentaerythrit (Fluka, purum); 181,7 g (0,8 Mol) 2-Brompropanoylbromid (Fluka, pract. 97%); 63,2 g (0,8 Mol) Pyridin (Fluka, puriss. p.a.); 500 ml THF (Fluka, puriss. p.a.); 1500 ml Sulfierkolben mit Rückflusskühler und mechanischem Rührwerk.

Man verfährt analog Beispiel 1. Das Rohprodukt (150 mg) wird durch Umkristallisieren in Isopropanol gereinigt. Man erhält 35,48 g (26%) des reinen Produkts. Schmelzpunkt: 95°C;

| Elementaranalyse: | | |
|---|---|---|
| C | H | Br |
| 30,21¹ | 3,58¹ | 47,30¹ |
| 30,70² | 3,61² | 45,28² |

| | | |
|---|---|---|
| ¹⁾berechnet; | | |
| ²⁾gefunden | | |

### Beispiel 4

Herstellung der Verbindung

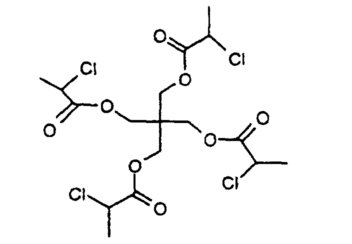

Ansatz:
2,72 g (0,02 Mol) Pentaerythrit (Fluka, purum); 10,15 g (0,08 Mol) 2-Chlorpropanoylchlorid (Fluka, pract. 97%); 6,32 g (0,08 Mol) Pyridin (Fluka, puriss. p.a.); 50 ml THF (Fluka, puriss. p.a.); 100 ml Sulfierkolben mit Rückflusskühler und mechanischem Rührwerk.

Analog Beispiel 3 (1/10 Ansatz) erhält man 6,10 g (48 %) des reinen Produkts; Schmelzpunkt: 84°C.

| Elementaranalyse: | | |
|---|---|---|
| C | H | Cl |
| 40,72¹ | 4,82¹ | 28,28¹ |
| 40,98² | 4,60² | 28,45² |

| | | |
|---|---|---|
| ¹⁾berechnet; | | |
| ²⁾gefunden | | |

### Beispiel 5:

### a) Herstellung eines "3-Stern"-Polymerisats mit niedrigem Molekulargewicht

Ansatz:
30,76 g (0,24 Mol) n-Butylacrylat (Fluka, purum); 0,57 g (4,0 mmol) Cu(I)Br (Fluka, purum mit Essigsäure gewaschen und getrocknet); 0,52 g (3,0 mmol) N,N,N',N",N"-Pentamethyldiethylentriamin (Fluka, purum); 10,78 g (20,0 mmol) 1-(2-Brompropionyloxy)-2-bis-(2-brompropionyloxymethyl)-butan (Initiator Beispiel 1); 30,76 g Dioxan (Fluka, puriss. p.a.); 150 ml Sulfierkolben mit Rückflusskühler, mechanischer Rührer und Tropftrichter; Anschlüsse für Vakuum und N₂.

Cu(I)Br und das Monomer n-Butylacrylat werden im Reaktionsgefäss eingewogen, 20 g Dioxan zugegeben und dieses mehrmals durch Evakuieren und Spülen mit N₂ entgast. Man gibt den Ligandenbildner PMDETA (N,N,N',N",N"-Pentamethyldiethylentriamin) zu, evakuiert und spült erneut mit N₂. Durch Eintauchen in ein Ölbad (85°C) erhitzt man die Mischung auf 50°C, bei welcher Temperatur man den Initiator (Beispiel 1), gelöst in 10,76 g Dioxan, aus dem Tropftrichter rasch zugibt. Die stark exotherme Polymerisation startet bei ca. 85°C bei rasch steigender Temperatur. Durch Kühlen mittels Eisbad wird die Temperatur auf 95-100°C gehalten. Nach 20 Min. Polymerisationszeit wird ein Umsatz von 100% (¹H-NMR-Kontrolle) erreicht, die Reaktionsmischung abgekühlt und mit 50 ml Dioxan verdünnt. Man gibt 30 g Al₂O₃ (Alox®) zu, rührt 1 h und filtriert. Die Polymerlösung wird im Rotationsverdampfer im Vakuum bei 80°C vollständig eingeengt. Ausbeute: 39,5 g (95%).

GPC (THF, PS-Standards): Mₙ=1700 (berechnet: 2080), M_{w}=2160, PDI=1,27; MALDI-TOF MS: Mₙ=2030, M_{w}=2300, PDI=1,17;

| Elementaranalyse: | | |
|---|---|---|
| C | H | Br |
| 57,27¹ | 8,07¹ | 11,54¹ |
| 57,26² | 8,26² | 11,44² |

| | | |
|---|---|---|
| ¹⁾berechnet; | | |
| ²⁾gefunden | | |

### b) Austausch der Br-Endgruppen durch 4-Benzoyloxy-TEMPO

Ansatz:
5,0 g (12,0 mmol Br-Endgruppen) Br-substituiertes Polymeres Beispiel 5 a); 3,31 g (12.0 mmol) 4-Benzoyloxy-TEMPO; 0,86 g (6,0 mmol) CuBr; 2,07 g (12,0 mmol) PMDETA; 7,5 ml Dioxan

In einem 25 ml Dreihalskolben mit Magnetrührer werden unter N₂ die genannten Reagenzien gemischt (ohne den Ligandenbildner PMDETA) und der Sauerstoff durch dreimaliges Evakuieren und Spülen mit N₂ ausgetrieben. Man gibt nun bei Raumtemperatur den Ligandenbildner PMDETA hinzu und heizt in einem Ölbad auf 65°C, wobei die Mischung sich rasch von orange über schwarz nach grün verfärbt. Man lässt noch 4 h bei 65°C reagieren und filtriert. Danach gibt man 10 ml Dioxan und viermal je 5 g Aluminiumoxid zu (zur Adsorption des restlichen Cu-Komplexes), rührt jeweils um und filtriert jeweils durch eine Nutsche. Die Lösung wird im Rotationsverdampfer bei 60°C 2 Stunden lang eingeengt. Man erhält 6,6 g (90%) Produkt.

GPC: Mₙ= 2172 (berechnet: 2290); M_{w}= 2600; PDI= 1,20; N-Gehalt: 2.48%; Br-Gehalt: <0,3%, woraus sich ein Austauschgrad höher als 97,4% errechnet.

### c) Herstellung eines "3-Stern"-Polymerisats mit höherem Molekulargewicht

Ansatz:
17,96 g (0,14 Mol) n-Butylacrylat (Fluka, purum); 0,111 g (0,8 mmol) Cu(I)Br (Fluka, purum mit Essigsäure gewaschen und getrocknet); 0,139 g (0,8 mmol) N,N,N',N",N"-Pentamethyldiethylentriamin (Fluka, purum); 2,10 g (3,9 mmol) 1-(2-Brompropionyloxy)-2-bis-(2-brompropionyloxymethyl)-butan (Initiator Beispiel 1); 30,76 g Dioxan (Fluka, puriss. p.a.); 150 ml Sulfierkolben mit Rückflusskühler, mechanischem Rührer und Tropftrichter; Anschlüsse für Vakuum und N₂.

Cu(I)Br und 14,0 g des Monomeren n-Butylacrylat werden im Reaktionsgefäss eingewogen und dieses mehrmals durch Evakuieren und Spülen mit N₂ entgast. Man gibt den Ligandenbildner (N,N,N',N",N"-Pentamethyldiethylentriamin) zu, evakuiert und spült erneut mit N₂. Durch Eintauchen in ein Ölbad (85°C) erhitzt man die Mischung auf 50°C, bei welcher Temperatur man den Initiator gelöst in 3,96 g restlichem Monomer aus dem Tropftrichter rasch zugibt. Die stark exotherme Polymerisation startet bei ca. 85°C bei rasch steigender Temperatur. Durch Kühlen mittels Eisbad wird die Temperatur auf maximal 105°C gehalten. Nach 45 Min. Polymerisationszeit wird ein Umsatz von 100% (¹H-NMR-Kontrolle) erreicht, die Reaktionsmischung abgekühlt und mit 50 ml Dioxan verdünnt. Man gibt 30 g Alox® zu, rührt 1 h und filtriert. Die Polymerlösung wird im Rotationsverdampfer im Vakuum bei 80°C vollständig eingeengt. Ausbeute: 18,5 g (92%).

GPC (THF, PS-Standards): Mₙ=4890 (berechnet: Mₙ=5150), M_{w}=6520, PDI=1,33;

| Elementaranalyse: | | |
|---|---|---|
| C | H | Br |
| 62,23¹ | 8,90¹ | 4,65¹ |
| 62,25² | 8,84² | 4,43² |

| | | |
|---|---|---|
| ¹⁾berechnet; | | |
| ²⁾gefunden | | |

### d) Herstellung eines "3-Stern"-Copolymerisats mit 1. Block Poly(n-BA) und 2. Block Poly-DMAEA

Ansatz:
5,15 g "3-Stern"-Polymerisat mit niedrigem Molekulargewicht, Beispiel 5 a); 0,71 g (5 mmol) 2-Dimethylaminoethylacrylat (DMAEA, BASF, techn.); 72,0 mg (0,5 mmol) Cu(I)Br (Fluka, purum mit Essigsäure gewaschen und getrocknet); 87,0 mg (0,5 mmol) N,N,N',N",N"-Pentamethyldiethylentriamin (Fluka, purum); 25 ml Rundkolben mit Magnetrührer und Septum.

Cu(I)Br, Das "3-Stern" Polymerisat mit niedrigem Molekulargewicht, Beispiel 5 a), und DMAEA werden im Reaktionsgefäss eingewogen und dieses mehrmals durch Evakuieren und Spülen mit N₂ entgast. Man gibt den Ligandenbildner PMDETA hinzu, evakuiert und spült erneut mit N₂. Durch Eintauchen in ein Ölbad erhitzt man die Mischung auf 50°C und lässt 30 Min. reagieren, wobei ein Umsatz von ca. 100% (¹H-NMR-Kontrolle) erreicht wird. Man kühlt ab, verdünnt mit 20 ml Ethylacetat und gibt 5 g Al₂O₃ (Alox®) zu, rührt 30 Min. und filtriert. Die Polymerlösung wird im Rotationsverdampfer bei 80°C vollständig eingeengt (1 h). Man erhält 5,0 g (85%) Produkt.

GPC (THF, PS-Standards): Mₙ=5590 (berechnet: Mₙ=5870), M_{w}=7520, PDI=1.35

| Elementaranalyse: | | | |
|---|---|---|---|
| C | H | N | Br |
| 61,80¹ | 8,93¹ | 1,19¹ | 4,08¹ |
| 62,24² | 8,80² | 0,87² | 3,44 |

| | | | |
|---|---|---|---|
| ¹⁾berechnet; | | | |
| ²⁾gefunden | | | |

### e) Herstellung eines "3-Stern"-Copolymerisats mit 1. Block Poly(n-BA) und 2. Block Poly-HEA

Ansatz:
5,15 g "3-Stern"-Polymerisat mit niedrigem Molekulargewicht, Beispiel 5 a); 0,58 g (5 mmol) 2-Hydroxyethylacrylat (DMAEA, BASF, techn.); 72,0 mg (0,5 mmol) Cu(I)Br (Fluka, purum mit Essigsäure gewaschen und getrocknet); 87,0 mg (0,5 mmol) N,N,N',N",N"-Pentamethyldiethylentriamin (Fluka, purum); 25 ml Rundkolben mit Magnetrührer und Septum.

Analog Beispiel 5 d) erhält man 5,0 g (85%) Produkt.

GPC (THF, PS-Standards): Mₙ=6530 (berechnet: Mₙ=5730), M_{w}=9690, PDI=1,48;

| Elementaranalyse: | | |
|---|---|---|
| C | H | Br |
| 61,17¹ | 8,70¹ | 4,18¹ |
| 61,67² | 8,83² | 3,42² |

| | | |
|---|---|---|
| ¹⁾berechnet; | | |
| ²⁾gefunden | | |

### Beispiel 6:

### a) Herstellung eines "4-Stern" Polymerisats mit niedrigem Molekulargewicht

Ansatz:
15,38 g (0,12 Mol) n-Butylacrylat (Fluka, purum); 0,28 g (2,0 mmol) Cu(I)Br (Fluka, purum mit Essigsäure gewaschen und getrocknet); 0,35 g (2,0 mmol) N,N,N',N",N"-Pentamethyldiethylentriamin (Fluka, purum); 6,76 g (20,0 mmol) 1,2,2,3-tetrakis(2-Brompropionyloxymethyl)-propan (Initiator Beispiel 3); 15,38 g Dioxan (Fluka, puriss. p.a.); 50 ml Sulfierkolben mit Rückflusskühler, mechanischem Rührer und Tropftrichter; Anschlüsse für Vakuum und N₂.

Analog Beispiel 5 a) lässt man bei 90°C reagieren, wobei man nach 90 Min. Polymerisationszeit einen Umsatz von ca. 100% (¹H-NMR-Kontrolle) erhält. Man isoliert 19,9 g (90%) des reinen Produkts.

GPC (THF, PS-Standards): Mₙ= 1770, M_{w}= 2080, PDI= 1,17 (berechnet: Mₙ= 2210); MALDI-TOF MS: Mₙ= 1920, M_{w}= 2020, PDI= 1,09;

| Elementaranalyse: | | |
|---|---|---|
| C | H | Br |
| 54,79¹ | 7,65¹ | 14,44¹ |
| 55,29² | 7,58² | 13,26² |

| | | |
|---|---|---|
| ¹⁾berechnet; | | |
| ²⁾gefunden | | |

### b) Austausch der Br-Endgruppen durch 4-Hydroxy-TEMPO

Ansatz:
5,0 g (8,3 mmol Br-Endgruppen) Br-substituiertes Polymeres Beispiel 6 a); 1,43 g (8,3 mmol) 4-Hydroxy-TEMPO; 1,20 g (8,3 mmol) CuBr; 1,91 g (8,3 mmol) Me₆TREN der Formel

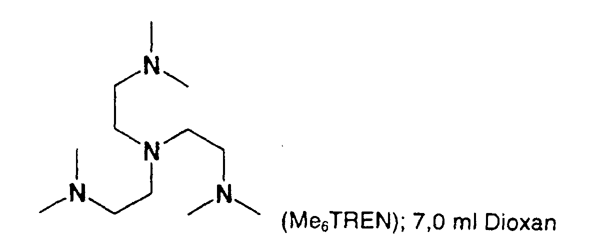

In einem 25 ml Dreihalskolben mit Magnetrührer werden unter N₂ die genannten Reagenzien gemischt (ohne den Ligandenbildner Me₆TREN) und der Sauerstoff durch dreimaliges Evakuieren und Spülen mit N₂ ausgetrieben. Man gibt nun bei Raumtemperatur den Ligandenbildner Me₆TREN zu, wobei die Mischung sich rasch von orange über schwarz nach grün verfärbt und eine Temperaturerhöhung bis 50°C stattfindet. Man lässt noch 1 h bei Raumtemperatur reagieren und filtriert. Danach gibt man 10 ml Dioxan und zweimal je 5,0 g Aluminiumoxid zu (zur Adsorption des restlichen Cu-Komplexes), rührt um und filtriert durch eine Nutsche. Die Lösung wird im Rotationsverdampfer bei 60°C 2 Stunden lang eingeengt. Man erhält 5,2 g (90%) Produkt.

GPC: Mₙ= 2280 (berechnet: 2140), M_{w}= 2630, PDI= 1.15

| Elementaranalyse: | | | |
|---|---|---|---|
| C | H | N | Br |
| 63,70¹ | 9,36¹ | 2,17¹ | 0,00¹ |
| 62,45² | 9,21² | 1,72² | 1,13 |

| | | | |
|---|---|---|---|
| ¹⁾berechnet; | | | |
| ²⁾gefunden | | | |

Aus dem Br-Gehalt lässt sich ein Austauschgrad von 91,5% errechnen.

### c) Herstellung eines "4-Stern"-Polymerisats mit höherem Molekulargewicht

Ansatz:
269,4 g (2,1 Mol) n-Butylacrylat (Fluka, purum); 1,67 g (12,0 mmol) Cu(I)Br (Fluka, purum mit Essigsäure gewaschen und getrocknet); 2,09 g (3,0 mmol) N,N,N',N",N"-Pentamethyldiethylentriamin (Fluka, purum); 39,54 g (20,0 mmol) 1,2,2,3-tetrakis(2-Brompropionyloxymethyl)-propan (Initiator Beispiel 3); 1000 ml Sulfierkolben mit Rückflusskühler, mechanisches Rührwerk und Tropftrichter; Anschlüsse für Vakuum und N₂.

Analog Beispiel 5 c) erhält man nach 45 Minuten Polymerisationszeit einen Umsatz von ca. 100% (¹H-NMR-Kontrolle) und isoliert nach Behandlung mit Al₂O₃ in Essigester 297,0 g (96%) des reinen Produkts.

GPC (THF, PS-Standards): Mₙ=5080 (berechnet: Mₙ= 5290), M_{w}= 6190, PDI= 1,22

| Elementaranalyse: | | |
|---|---|---|
| C | H | Br |
| 61,07¹ | 8,68¹ | 6,04¹ |
| 61,10² | 8,63² | 5,56² |

| | | |
|---|---|---|
| ¹⁾berechnet; | | |
| ²⁾gefunden | | |

### d) Herstellung eines "4-Stern"-Copolymerisats mit 1. Block Poly(n-BA) und 2. Block Poly-DMAEA

Ansatz:
80,0 g "4-Stern" Polymerisat mit niedrigem Molekulargewicht, Beispiel 6 a); 10,74 g (75 mmol) 2-Dimethylaminoethylacrylat (DMAEA, BASF, techn.); 1,08 g (7,5 mmol) Cu(I)Br (Fluka, purum mit Essigsäure gewaschen und getrocknet); 1,23 g (7,5 mmol) N,N,N',N",N"-Pentamethyldiethylentriamin (Fluka, purum); 750 ml Sulfierkolben mit mechanischem Rührwerk und Septum.

Cu(I)Br, das "4-Stern" Polymerisat mit niedrigem Molekulargewicht, Beispiel 6 a), und DMAEA werden im Reaktionsgefäss eingewogen und dieses mehrmals durch Evakuieren und Spülen mit N₂ entgast. Man gibt den Ligandenbildner (N,N,N',N",N"-Pentamethyldiethylentriamin) hinzu, evakuiert und spült erneut mit N₂. Durch Eintauchen in ein Ölbad erhitzt man die Mischung auf 90°C und lässt 60 Min. reagieren, wobei ein Umsatz von ca. 100% (¹H-NMR-Kontrolle) erreicht wird. Man kühlt ab, verdünnt mit 150 ml Ethylacetat und gibt 80,0 g Al₂O₃ (Alox®) zu, rührt 60 Min. und filtriert. Die Polymerlösung wird im Rotationsverdampfer bei 80°C vollständig eingeengt (1 h). Man erhält 76,6 g (85%) Produkt.

GPC (THF, PS-Standards): Mₙ=5820 (berechnet: Mₙ=5800), M_{w}=7410, PDI=1.27

| Elementaranalyse: | | | |
|---|---|---|---|
| C | H | N | Br |
| 60,79¹ | 8,74¹ | 1,17¹ | 5,33¹ |
| 61,36² | 8,86² | 1,04² | 4,16² |

| | | | |
|---|---|---|---|
| ¹⁾berechnet; | | | |
| ²⁾gefunden | | | |

### e) Herstellung eines "4-Stern" Copolymerisats mit 1. Block Poly(n-BA) und 2. Block Poly-HEA

Ansatz:
80,0 g "4-Stern" Polymerisat mit niedrigem Molekulargewicht, Beispiel 6 a); 8,71 g (75 mmol) 2-Hydroxyethylacrylat (HEA, BASF, techn.); 1,08 g (7,5 mmol) Cu(I)Br (Fluka, purum mit Essigsäure gewaschen und getrocknet); 1,23 g (7,5 mmol) N,N,N',N",N"-Pentamethyldiethylentriamin (Fluka, purum); 750 ml Sulfierkolben mit mechanischem Rührwerk und Septum.

Analog Beispiel 6 d) erhält man 68,4 g (77%) Produkt.

GPC (THF, PS-Standards): Mₙ= 6880 (berechnet: Mₙ= 5660), M_{w}= 9730, PDI= 1,41

| Elementaranalyse: | | |
|---|---|---|
| C | H | Br |
| 60,15¹ | 8,51¹ | 5,45¹ |
| 60,96² | 8,66² | 4,27² |

| | | |
|---|---|---|
| ¹⁾berechnet; | | |
| ²⁾gefunden | | |

### Beispiel 7

Herstellung von 1,3,5-tris(2-Brom-2-Methylpropanoyloxy)-benzol:

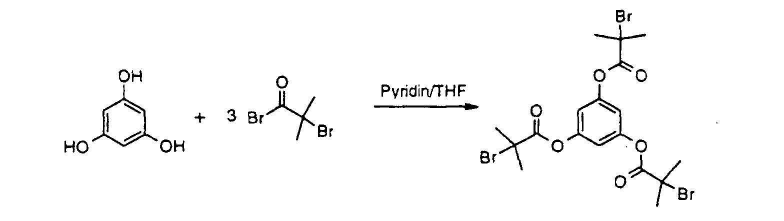

In einem 100 ml Rundkolben werden unter Rühren mit einem Magnetrührer 5,0 g (39,6 mmol) 1,3,5-Trihydroxybenzol (Fluka, puriss) in 40 ml THF gelöst and 9,40 g (118,8 mmol) Pyridin (Fluka, puriss) hinzugesetzt. Man kühlt die Lösung auf 5°C ab und fügt langsam unter Rühren während einer Stunde 27,34 g (118,8 mmol) in 20 ml THF gelöste α-Brom-isobuttersäurebromid (Fluka pract) hinzu. Nach der Zugabe rührt man noch eine Stunde lang bei 60°C weiter, lässt die Suspension auf Raumtemperatur abkühlen und filtriert diese. Man dampft das Lösungsmittel im Rotationsverdampfer ab, wäscht den Rückstand mit Wasser und kristallisiert diesen aus Isopropanol um. Ausbeute: 11,04 g (48,2%) weisse Kristalle. Schmelzpunkt des dünnschichtchromatographisch gereinigten Produkts: 186.4°C.

| Elementaranalyse: | | |
|---|---|---|
| C | H | Br |
| 37,72¹ | 3,69¹ | 41,82¹ |
| 38,10² | 3,56² | 41,51² |

| | | |
|---|---|---|
| ¹⁾berechnet; | | |
| ²⁾gefunden | | |

### Beispiel 8

Herstellung von 1,2,3,4,5,6-Hexakis(2-chlor-propanoyloxy)-n-hexan

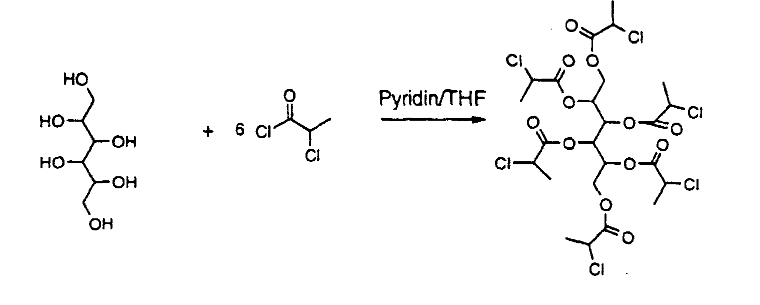

In einem 100 ml Rundkolben werden unter Rühren mit einem Magnetrührer 5,0 g (27.4 mmol) Sorbit (Fluka, puriss) in 10 ml THF gelöst and 13.0 g (164.4 mmol) Pyridin (Fluka, puriss) hinzugesetzt. Man kühlt die Lösung auf Raumtemperatur ab und fügt langsam unter Rühren während einer Stunde 23,35 g (164,4 mmol) in 20 ml THF gelöste 2-Chlorpropanoylchlorid (Fluka, pract.) hinzu. Nach der Zugabe rührt man noch vier Stunden lang bei 60°C weiter, lässt die Suspension auf Raumtemperatur abkühlen und filtriert diese. Man dampft das Lösungsmittel im Rotationsverdampfer ab, löst den Rückstand in tert.-Butylmethylether, wäscht mit Wasser und filtriert über Aktivkohle. Das Lösungsmittel wird im Vakuum bei 0,05 mbar entfernt. Ausbeute: 11,85 g (59,6%) eines gelblichen Öls. Dieses wird dann noch säulenchromatographisch (Methode "Flash") über Silicagel gereinigt. Ausbeute des dünnschichtchromatographisch gereinigten Produkts: 7,02 g (35.3%).

| Elementaranalyse: | | |
|---|---|---|
| C | H | Cl |
| 39,73¹ | 4,45¹ | 29,36¹ |
| 40,32² | 4,31² | 29,11² |

| | | |
|---|---|---|
| ¹⁾berechnet; | | |
| ²⁾gefunden | | |

### Beispiel 9

### Herstellung eines "3-Stern" Polymerisats mit höherem Molekulargewicht

In einem 25 ml Rundkolben, der mit Septum und Magnetrührer versehen ist, werden 4,71 g (47 mmol) Methylmethacrylat (MMA, Fluka puriss) unter Stickstoffatmosphäre wie folgt polymerisiert: Man gibt die jeweiligen Mengen Cu(I)Br-Katalysator (Fluka, purum), den Initiator 1,3,5-tris(2-Brom-2-Methylpropanoyloxy)-benzol, Beispiel 7, das Lösungsmittel (falls erforderlich) and MMA in den Kolben, den man mit einem Gummiseptum fest verschliesst. Unter Rühren evakuiert man das Gefäss und spült dieses dreimal mit Stickstoff. Anschliessend gibt man den Ligandenbildner PMDETA (N,N,N',N",N"-Pentamethyldiethylentriamin, Fluka, purum) mit einer Spritze hinzu. Man erhitzt das Gefäss in einem Ölbad auf 90°C und beobachtet den Fortgang der Reaktion durch regelmässige Probenentnahme und NMR-Kontrolle in CDCl₃. In der Tabelle 1 sind die Reaktionsbedingungen beschrieben:

**Tabelle 1**

| Nr. | Initiator (mg) | Cu(I)Br (mg) | PMDETA (mg) | Lösungsmittel | Reaktionszeit (h) | Umsatz (%) | Mₙ (ber.) |
|---|---|---|---|---|---|---|---|
| 1 | 67 | 34 | 41 | - | 0,33 | 69 | 28100 |
| 2 | 22,3 | 11,2 | 13,5 | - | 3,0 | 60 | 72500 |
| 3 | 13,5 | 6,7 | 8,1 | 3 ml Dioxan | 5,5 | 50 | 101000 |

Aufarbeitung: Nach Aufnahme in 20 ml Essigester und Filtrieren fällt man das Polymer aus150 ml Ethanol aus. Das Polymerisat (poly(MMA)) erhält man als weisses Pulver nach Filtrieren und Trockenen im Vakuum bei 50°C. Tab. 2 enthält Angaben über die Ausbeute und charakteristische Daten wie Molekulargewichtsbestimmungen mittels GPC (THF, PS-Standard) und Lichtstreuung (LS, Wyatt Down DSP: "Multi Angle Laser Light Scattering Instrument").

**Tabelle 2**

| Nr. | Ausbeute (%) | GPC: Mₙ | GPC: M_{w} | GPC: PDI | LS: M_{w} | LS: PDI |
|---|---|---|---|---|---|---|
| 1 | 68,6 | 50200 | 67000 | 1,33 | 82500 | 1,29 |
| 2 | 40,3 | 76500 | 95200 | 1,24 | 115700 | 1,19 |
| 3 | 36,1 | 87700 | 108000 | 1.,23 | 124400 | 1,18 |

Die PDI-Werte sind tief und der höhere M_{w}-Wert der LS relativ zu jenem der GPC deutet auf eine kompakte Molekülstruktur dieser sternförmigen Makromoleküle hin.

### Beispiel 10

Herstellung eines sechsfach verzweigten Polymerisats mit höherem Molekulargewicht In einem 25 ml Rundkolben, der mit Septum und Magnetrührer versehen ist, werden 4.71 g (47 mmol) n-Butylacrylat (n-BA, Fluka puriss) unter Stickstoffatmosphäre wie folgt polymerisiert: Man gibt die jeweiligen Mengen Cu(I)Br-Katalysator (Fluka, purum), den Initiator 1,2,3,4,5,6-Hexakis(2-chlor-propanoyloxy)-n-hexan, Beispiel 8, das Lösungsmittel (falls erforderlich) and n-BA in den Kolben, den man mit einem Gummiseptum fest verschliesst. Unter Rühren evakuiert man das Gefäss und spült dieses dreimal mit Stickstoff. Anschliessend gibt man den Ligandenbildner PMDETA (N,N,N',N",N"-Pentamethyldiethylentriamin, Fluka, purum) mit einer Spritze hinzu. Man erhitzt das Gefäss in einem Ölbad auf 90°C und beobachtet den Fortgang der Reaktion durch regelmässige Probenentnahme und NMR-Kontrolle in CDCl₃. In der Tabelle 3 sind die Reaktionsbedingungen beschrieben:

**Tabelle 3**

| Nr. | Initiator (mg) | Cu(I)Cl (mg) | PMDETA (mg) | Reaktionszeit(h) | Umsatz (%) | Mₙ (calc) |
|---|---|---|---|---|---|---|
| 1 | 85 | 23,2 | 41 | 1,25 | 90 | 46800 |
| 2 | 34,1 | 9,3 | 16,3 | 1,5 | 87 | 112000 |
| 3 | 167 | 22,8 | 39,9 | 0,58 | 90 | 23800 |

Aufarbeitung: Nach Verdünnung des Reaktionsgemisches mit 25 ml Essigester und Versetzen mit 1,5 g Al₂O₃ (Adsorption des Katalysators), Filtrieren und Trocknen bei 100°C im Vakuum (1 h, < 0.4 mbar) erhält man Poly(n-BA) als Öl. Tab. 3 enthält Angaben über die Ausbeute und charakteristische Daten wie Molekulargewichtsbestimmungen mittels GPC (THF, PS-Standard) und Lichtstreuung (LS, Wyatt Down DSP: "Multi Angle Laser Light Scattering Instrument").

**Tabelle 4**

| Nr. | Ausbeute (%) | GPC: Mₙ | GPC: M_{w} | GPC: PDI | LS: M_{w} | LS: PDI |
|---|---|---|---|---|---|---|
| 1 | 84.7 | 38700 | 47100 | 1.22 | 49400 | 1.17 |
| 2 | 87 | 98400 | 135300 | 1.32 | 146600 | 1.28 |
| 3 | 90 | 24000 | 33800 | 1.41 | 39400 | 1.32 |

Die PDI-Werte sind tief und der höhere M_{w}-Wert der LS relativ zu jenem der GPC deutet auf eine kompakte Molekülstruktur dieser stemförmigen Makromoleküle hin.

### Beispiel 11

Analog Beispiel 3 wird der Initiator 1,2,2,3-Tetrakis(2-bromopropionyloxymethyl)-propan hergestellt. n-Butylacrylat (n-BA) wird mit diesem Initiator analog Beispiel 5a zum 4-armigen sternförmigen Polymer umgesetzt: Charakterisierung: Mₙ=5080, M_{w}=6200, PDI=1.22, Br (gefunden): 5,56%.

80,0 g dieses 4-armigen sternförmigen Poly-n-butylacrylates und 1,08 g (7,5 mmol) CuBr (Fluka, gereinigt durch Waschen mit Essigsäure) werden in einen 750 ml Rundkolben mit mechanischem Rührer gegeben. Die Luft wird durch Rühren und dreimaliges Evakuieren und Spülen mit Stickstoff ausgetrieben. 1,23 g (1,57 ml, 7,5 mmol) PMDETA (Fluka/purum) werden mit einer Spritze durch ein Septum hinzugegeben. Das Gefäss wird und erneut evakuiert und mit Stickstoff gespült. Wenn die Mischung durch Rühren homogen wird, wird diese auf einem Oelbad auf 60°C erhitzt. 10,74 g (11,47 ml, 75 mmol) 2-Dimethylaminoethylacrylat (BASF, technische Qualität) werden mit einer Spritze durch das Septum hinzugefügt. Die Temperatur wird auf 90°C während einer Stunde (Polymerisationszeit) erhöht. Der Umsatz wird durch ¹H-NMR-Analyse in CDCl₃ zu ca. 100 % bestimmt. Nach Abkühlen auf Raumtemperatur werden 150 ml Ethylacetat und 80 g neutrales Aluminumoxid (Alox® für Chromatographie) zugegeben. Das Polymer wird nach 1 h Rühren bei Raumtemperatur, Filtration und einstündigem Trocknen im Rotationsverdampfer bei 80°C im Hochvakuum erhalten. Ausbeute: 76,62 g (85 %).

| Elementaranalyse: | | | | |
|---|---|---|---|---|
| | C | H | N | Br |
| berechnet | 60,79 | 8,74 | 1,17 | 5,33 |
| gefunden | 61,36 | 8,86 | 1,04 | 4,16 |

Cu: 166 ppm (X-ray Fluoreszenz); GPC (THF): Mₙ = 5800,
M_{w} = 7370, PDI = 1,27.

## Patentansprüche

1. Ein Polymer oder Blockcopolymer der Formel: 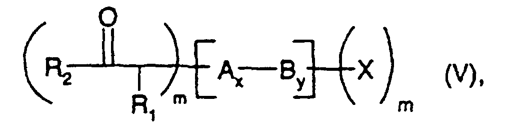 worin
R₁ Wasserstoff, C₁-C₄-Alkyl, Cyan, Phenyl oder C₁-C₄-Alkylphenyl;
R₂ den Rest eines acylierten, verzweigten, dreiwertigen Alkohols, den Rest eines vollständig oder partiell acylierten, linearen oder verzweigten, vierwertigen Alkohols, den Rest eines vollständig oder partiell acylierten, linearen, fünf- oder sechswertigen Alkohols, den Rest einer vollständig oder partiell acylierten, linearen oder cyclischen C₄-C₆-Aldose oder C₄-C₆-Ketose oder den Rest eines vollständig oder partiell acylierten Disaccharids;
A und B Polymerblöcke aus ethylenisch ungesättigten Monomereneinheiten;
x und y die Anzahl der Monomereneinheiten in den Blöcken A und B, wobei ein Wert von x und y null und der andere Wert ganze Zahlen grösser als null oder beide Werte x und y Zahlen grösser als null betragen;
X Chlor, Brom oder lod; und
m ganze Zahlen von drei bis sechs bedeuten.

2. Ein Blockcopolymer (V) gemäss Anspruch 1, worin
R₁ C₁-C₃-Alkyl oder Phenyl;
X Chlor oder Brom und
R₂ den Rest eines acylierten, verzweigten, dreiwertigen Alkohols, den Rest eines acylierten, linearen oder verzweigten, vierwertigen Alkohols oder den Rest eines vollständig oder partiell acylierten, linearen, fünf- oder sechswertigen Alkohols,
A und B Polymerblöcke aus ethylenisch ungesättigten Monomereneinheiten;
x und y ganze Zahlen grösser als null und die Anzahl der Monomereneinheiten in den Blöcken A und B darstellen; und
m drei oder vier bedeuten.

3. Ein Polymer oder Blockcopolymer der Formel: 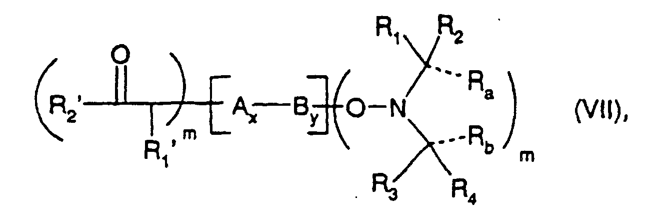 worin
R₁' Wasserstoff, C₁-C₄-Alkyl, Cyan, Phenyl oder C₁-C₄-Alkylphenyl;
R₂' den Rest eines acylierten, verzweigten, dreiwertigen Alkohols, den Rest eines vollständig oder partiell acylierten, linearen oder verzweigten, vierwertigen Alkohols, den Rest eines vollständig oder partiell acylierten, linearen, fünf- oder sechswertigen Alkohols, den Rest einer vollständig oder partiell acylierten, linearen oder cyclischen C₄-C₆-Aldose oder C₄-C₆-Ketose oder den Rest eines vollständig oder partiell acylierten Disaccharids;
A und B Polymerblöcke aus ethylenisch ungesättigten Monomereneinheiten;
x und y die Anzahl der Monomereneinheiten in den Blöcken A und B, wobei ein Wert von x und y null und der andere Wert ganze Zahlen grösser als null oder beide Werte x und y ganze Zahlen grösser als null betragen;
m ganze Zahlen von drei bis sechs bedeuten;
einer von R₁ und R₂ C₁-C₇-Alkyl und der andere C₁-C₄-Alkyl oder durch C₁-C₄-Alkoxycarbonyl oder C₁-C₄-Alkoxy substituiertes C₁-C₄-alkyl bedeutet; oder
R₁ und R₂ zusammen mit dem benachbarten C-Atom C₃-C₇-Cycloalkyl bedeuten;
R₃ und R₄ die Bedeutungen von R₁ und R₂ haben;
Rₐ C₁-C₄-Alkyl, Cyan, C₁-C₄-Alkoxycarbonyl, C₁-C₄-Alkanoyloxy, C₁-C₄-Alkanoyloxy-C₁-C₄-alkyl, Carbamoyl, Mono- oder Di-C₁-C₄-alkylcarbamoyl, Mono- oder Di-2-hydroxyethylcarbamoyl, Amidino, 2-Imidazolyl, 1-Hydroxy-2-hydroxymethyl-2-propylcarbamoyl, oder 1,1-Dihydroxymethyl-2-hydroxycarbamoyl; und
R_{b} die Bedeutungen von Rₐ hat; oder
Rₐ und R_{b} zusammen eine bivalente Gruppe und eine aliphatische oder aromatische heterocyclische Gruppe mit 5, 6, 7 oder 8-Ringgliedem bilden, welche 1-3 zusätzliche Heteroatome aus der Gruppe Stickstoff, Sauerstoff und Schwefel enthalten kann.

4. Eine Polymerzusammensetzung enthaltend ein Polymer oder Blockcopolymer (V) gemäss Anspruch 1, worin R₁, R₂, A, B, x, y und m die genannten Bedeutungen haben, und in Polymerzusammensetzungen übliche Zusatzstoffe.

5. Eine Polymerzusammensetzung enthaltend
a) ein Polymer oder Blockcopolymer (V) gemäss Anspruch 1, worin R₁, R₂, A, B, x, y und m die genannten Bedeutungen haben; und
b) ein weiteres Polymer oder Oligomer der Formel
Aₓ-B_{y} (IX),
worin
A und B Polymerblöcke aus ethylenisch ungesättigten Monomereneinheiten und x und y die Anzahl der Monomereneinheiten in den Blöcken A und B bedeuten, wobei ein Wert von x und y null und der andere Wert ganze Zahlen grösser als null oder beide Werte x und y Zahlen grösser als null betragen.

6. Verfahren zur Herstellung eines Polymeren oder Blockcopolymeren (V), worin R₁, R₂, A, B, X, x, y und m die in Anspruch 1 genannten Bedeutungen haben, **dadurch gekennzeichnet, dass** man durch radikalische Atomtransferpolymerisation (ATRP) in Gegenwart des α-Halogencarbonsäureesters (I) 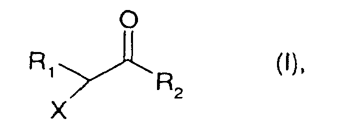 worin
R₁ Wasserstoff, C₁-C₄-Alkyl, Cyan, Phenyl oder C₁-C₄-Alkylphenyl;
X Chlor, Brom oder lod; und
R₂ den Rest eines acylierten, verzweigten, dreiwertigen Alkohols, den Rest eines vollständig oder partiell acylierten, linearen oder verzweigten, vierwertigen Alkohols, den Rest eines vollständig oder partiell acylierten, linearen, fünf- oder sechswertigen Alkohols, den Rest einer vollständig oder partiell acylierten, linearen oder cyclischen C₄-C₆-Aldose oder
C₄-C₆-Ketose oder den Rest eines vollständig oder partiell acylierten Disaccharids darstellen, als Polymerisationsinitiator und eines oxydierbaren Übergangsmetallkomplexkatalysators den Polymerblöcken A und B zugrundeliegende aliphatische Monomere mit Ethylengruppen einer Polymerisationsreaktion unterwirft.

7. Verwendung eines Polymeren oder Blockcopolymeren (V) zur Herstellung von Polymeren oder Blockcopolymeren, worin •X durch eine offenkettige oder cyclische Gruppe R'R"N-O• ersetzt ist.

## Claims

1. A polymer or block copolymer of formula: 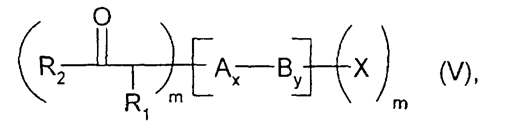 wherein
R₁ is hydrogen, C₁-C₄alkyl, cyano, phenyl or C₁-C₄alkylphenyl;
R₂ is the radical of an acylated, branched, trihydric alcohol, the radical of a fully or partially acylated, linear or branched, tetrahydric alcohol, the radical of a fully or partially acylated, linear, penta- or hexa-hydric alcohol, the radical of a fully or partially acylated, linear or cyclic C₄-C₆aldose or C₄-C₆ketose or the radical of a fully or partially acylated disaccharide;
A and B are polymer blocks of ethylenically unsaturated monomer units;
x and y denote the number of monomer units in the blocks A and B, one value of x and y being zero and the other value being an integer greater than zero, or both values x and y being integers greater than zero;
X is chlorine, bromine or iodine; and
m denotes an integer from three to six.

2. A block copolymer (V) according to claim 1, wherein
R₁ is C₁-C₃alkyl or phenyl;
X is chlorine or bromine and
R₂ is the radical of an acylated, branched, trihydric alcohol, the radical of an acylated, linear or branched, tetrahydric alcohol or the radical of a fully or partially acylated, linear, penta- or hexa-hydric alcohol,
A and B are polymer blocks of ethylenically unsaturated monomer units;
x and y denote integers greater than zero and represent the number of monomer units in the blocks A and B; and
m is three or four.

3. A polymer or block copolymer of formula: 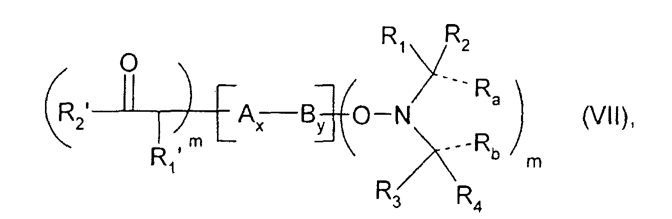 wherein
R₁' is hydrogen, C₁-C₄alkyl, cyano, phenyl or C₁-C₄alkylphenyl;
R₂' is the radical of an acylated, branched, trihydric alcohol, the radical of a fully or partially acylated, linear or branched, tetrahydric alcohol, the radical of a fully or partially acylated, linear, penta- or hexa-hydric alcohol, the radical of a fully or partially acylated, linear or cyclic C₄-C₆aldose or C₄-C₆ketose or the radical of a fully or partially acylated disaccharide;
A and B are polymer blocks of ethylenically unsaturated monomer units;
x and y denote the number of monomer units in the blocks A and B, one value of x and y being zero and the other value being an integer greater than zero, or both values x and y being integers greater than zero;
m denotes an integer from three to six;
one of R₁ and R₂ is C₁-C₇alkyl and the other is C₁-C₄alkyl or C₁-C₄alkyl substituted by C₁-C₄alkoxycarbonyl or by C₁-C₄alkoxy; or
R₁ and R₂ together with the adjacent carbon atom are C₃-C₇cycloalkyl;
R₃ and R₄ have the meanings of R₁ and R₂;
Rₐ is C₁-C₄alkyl, cyano, C₁-C₄alkoxycarbonyl, C₁-C₄alkanoyloxy, C₁-C₄alkanoyloxy-C₁-C₄-alkyl, carbamoyl, mono- or di-C₁-C₄alkylcarbamoyl, mono- or di-2-hydroxyethylcarbamoyl, amidino, 2-imidazolyl, 1-hydroxy-2-hydroxymethyl-2-propylcarbamoyl or 1,1-dihydroxymethyl-2-hydroxycarbamoyl; and
R_{b} has the meanings of Rₐ; or
Rₐ and R_{b} together form a bivalent group and an aliphatic or aromatic heterocyclic group having 5, 6, 7 or 8 ring members, which can contain from 1 to 3 additional hetero atoms from the group nitrogen, oxygen and sulfur.

4. A polymer composition comprising a polymer or block copolymer (V) according to claim 1, wherein R₁, R₂, A, B, x, y and m are as defined, and additives customary in polymer compositions.

5. A polymer composition comprising
a) a polymer or block copolymer (V) according to claim 1,
wherein R₁, R₂, A, B, x, y and m are as defined; and
b) a further polymer or oligomer of formula
Aₓ-By (IX),
wherein
A and B are polymer blocks of ethylenically unsaturated monomer units and x and y denote the number of monomer units in the blocks A and B, one value of x and y being zero and the other value being an integer greater than zero, or both values x and y being integers greater than zero.

6. A process for the preparation of a polymer or block copolymer (V), wherein R₁, R₂, A, B, X, x, y and m are as defined in claim 1, in which process ethylene-group-containing aliphatic monomers that form the basis of the polymer blocks A and B are subjected to a polymerisation reaction by atom transfer radical polymerisation (ATRP) in the presence of the α-halocarboxylic acid ester (I) 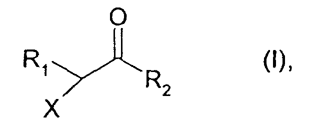 wherein
R₁ is hydrogen, C₁-C₄alkyl, cyano, phenyl or C₁-C₄alkylphenyl;
X is chlorine, bromine or iodine; and
R₂ is the radical of an acylated, branched, trihydric alcohol, the radical of a fully or partially acylated, linear or branched, tetrahydric alcohol, the radical of a fully or partially acylated, linear, penta- or hexa-hydric alcohol, the radical of a fully or partially acylated, linear or cyclic C₄-C₆aldose or C₄-C₆ketose or the radical of a fully or partially acylated disaccharide,
as polymerisation initiator and in the presence of an oxidisable transition metal complex catalyst.

7. Use of a polymer or block copolymer (V) in the preparation of a polymer or block copolymer wherein •X is replaced by an open-chain or cyclic group R'R"N-O•.

## Revendications

1. Polymère ou copolymère séquencé de formule : 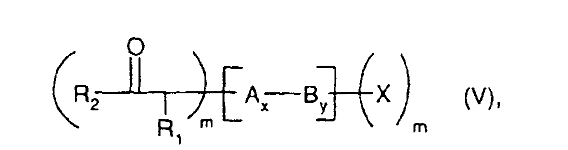 dans laquelle
R₁ représente un atome d'hydrogène, un groupe alkyle en C₁ à C₄, un groupe cyano, phényle ou (alkyle en C₁ à C₄)phényle ;
R₂ représente le radical d'un triol ramifié, acylé, le radical d'un tétraol linéaire ou ramifié, entièrement ou partiellement acylé, le radical d'un pentaol ou d'un hexaol linéaire entièrement ou partiellement acylé, le radical d'un aldose en C₄ à C₆ ou d'un cétose en C₄ à C₆ linéaire ou cyclique, entièrement ou partiellement acylé, ou le radical d'un disaccharide entièrement ou partiellement acylé ;
A et B représentent des séquences polymères de motifs monomères à insaturation éthylénique ;
x et y représentent le nombre de motifs monomères dans les séquences A et B, une valeur de x et y étant de zéro et l'autre valeur étant un nombre entier supérieur à zéro ou les deux valeurs de x et y étant un nombre entier supérieur à zéro ;
X représente un atome de chlore, de brome ou d'iode ;
et
m représente un nombre entier de trois à six.

2. Copolymère séquencé (V) selon la revendication 1, où
R₁ représente un groupe alkyle en C₁ à C₃ ou phényle ;
X représente un atome de chlore ou de brome et
R₂ représente le radical d'un triol ramifié, acylé, le radical d'un tétraol linéaire ou ramifié, acylé ou le radical d'un pentaol ou d'un hexaol linéaire, entièrement ou partiellement acylé ;
A et B représentent des séquences polymères de motifs monomères à insaturation éthylénique ;
x et y représentent un nombre entier supérieur à zéro et le nombre de motifs monomères dans les séquences A et B ; et
m vaut trois ou quatre.

3. Polymère ou copolymère séquencé de formule : 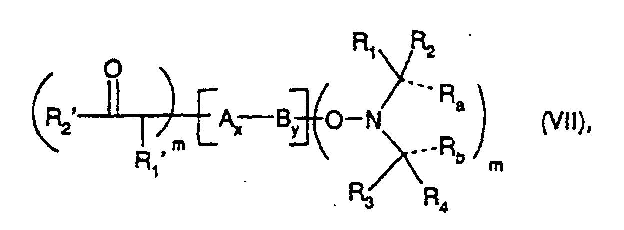 dans laquelle
R₁' représente un atome d'hydrogène, un groupe alkyle en C₁ à C₄, un groupe cyano, phényle ou (alkyle en C₁ à C₄)phényle ;
R₂' représente le radical d'un triol ramifié, acylé, le radical d'un tétraol linéaire ou ramifié, entièrement ou partiellement acylé, le radical d'un pentaol ou d'un hexaol linéaire entièrement ou partiellement acylé, le radical d'un aldose en C₄ à C₆ ou d'un cétose en C₄ à C₆ linéaire ou cyclique, entièrement ou partiellement acylé, ou le radical d'un disaccharide entièrement ou partiellement acylé ;
A et B représentent des séquences polymères de motifs monomères à insaturation éthylénique ;
x et y représentent le nombre de motifs monomères dans les séquences A et B, une valeur de x et y étant de zéro et l'autre valeur étant un nombre entier supérieur à zéro ou les deux valeurs de x et y étant un nombre entier supérieur à zéro ;
m représente un nombre entier de trois à six ;
l'un de R₁ et R₂ représente un groupe alkyle en C₁ à C₇ et l'autre un groupe alkyle en C₁ à C₄ ou alkyle en C₁ à C₄ substitué par un groupe (alcoxy en C₁ à C₄)carbonyle ou (alcoxy en C₁ à C₄) ; ou
R₁ et R₂ représentent, conjointement avec l'atome de C vicinal, un groupe cycloalkyle en C₃ à C₇ ;
R₃ et R₄ ont les significations de R₁ et R₂ ;
Rₐ représente un groupe alkyle en C₁ à C₄, cyano, (alcoxy en C₁ à C₄)carbonyle, (alcanoyle en C₁ à C₄)oxy, (alcanoyle en C₁ à C₄)oxy-(alkyle en C₁ à C₄), carbamoyle, mono- ou di-(alkyle en C₁ à C₄)carbamoyle, mono- ou di-2-hydroxyéthylcarbamoyle, amidino, 2-imidazolyle, 1-hydroxy-2-hydroxyméthyl-2-propylcarbamoyle ou 1,1-dihydroxyméthyl-2-hydroxycarbamoyle ; et
R_{b} a les significations de Rₐ ; ou
Rₐ et R_{b} représentent ensemble un groupe bivalent et un groupe hétérocyclique aliphatique ou aromatique à 5, 6, 7 ou 8 éléments dans le cycle, lequel peut contenir 1 à 3 hétéroatomes supplémentaires du groupe formé par l'azote, l'oxygène et le soufre.

4. Composition polymère contenant un polymère ou un copolymère séquencé (V) selon la revendication 1, où R₁, R₂, A, B, x, y et m ont les significations mentionnées, et les adjuvants habituels dans les compositions polymères.

5. Composition polymère contenant
a) un polymère ou un copolymère séquencé (V) selon la revendication 1, où
R₁, R₂, A, B, x, y et m ont les significations mentionnées ; et
b) un autre polymère ou oligomère de formule
Aₓ-B_{y} (IX),
dans laquelle
A et B représentent les séquences polymères issues de motifs monomères à insaturation éthylénique et x et y représentent le nombre de motifs monomères dans les séquences A et B, une valeur de x et y étant de zéro et l'autre valeur étant un nombre entier supérieur à zéro ou les deux valeurs de x et y étant un nombre entier supérieur à zéro.

6. Procédé de production d'un polymère ou d'un copolymère séquencé (V), où R₁, R₂, A, B, X, x, y et m ont les significations mentionnées dans la revendication 1, **caractérisé en ce que** l'on soumet des monomères aliphatiques contenant des groupes éthylène, qui sont à la base des séquences polymères A et B, à une réaction de polymérisation au moyen d'une polymérisation radicalaire par transfert d'atome (ATRP), en présence de l'ester d'acide α-halogénocarboxylique (I) 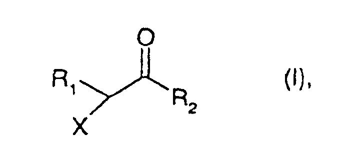 où
R₁ représente un atome d'hydrogène, un groupe alkyle en C₁ à C₄, un groupe cyano, phényle ou (alkyle en C₁ à C₄)phényle ;
X représente un atome de chlore, de brome ou d'iode ; et
R₂ représente le radical d'un triol ramifié, acylé, le radical d'un tétraol linéaire ou ramifié, entièrement ou partiellement acylé, le radical d'un pentaol ou d'un hexaol linéaire, entièrement ou partiellement acylé, le radical d'un aldose en C₄ à C₆ ou d'un cétose en C₄ à C₆ linéaire ou cyclique, entièrement ou partiellement acylé, ou le radical d'un disaccharide entièrement ou partiellement acylé, en tant qu'amorceur de polymérisation, et d'un catalyseur de complexe de métal de transition oxydable.

7. Utilisation d'un polymère ou d'un copolymère séquencé (V) pour la production de polymères ou de copolymères séquencés, où •X est substitué par un groupe R'R"N-O• à chaîne ouverte ou cyclique.
